# EUROPÄISCHE PATENTSCHRIFT

(11) **EP 0 640 607 B1**
(45) Veröffentlichungstag und Bekanntmachung des Hinweises auf die Patenterteilung: **05.02.1997**
(21) Anmeldenummer: 94112622.9
(22) Anmeldetag: 12.08.1994
(51) Int. Cl.: C07D 499/86, A61K 31/43

(54) **2-Beta-Alkenyl-penem-Sulfonverbindungen als beta- Lactamase-Hemmer**
2-Beta-alkenyl-penem-sulfonyl compounds as beta-lacterase inhibitors
2-Bêta-alcényl pénem sulfonyl composés en tant qu'inhibiteurs de bêta-lactamase

(30) Priorität: 24.08.1993 CH 2511/93; 31.05.1994 CH 1687/94
(43) Veröffentlichungstag der Anmeldung: 01.03.1995
(73) Patentinhaber: F. HOFFMANN-LA ROCHE AG, 4002 Basel (CH)
(72) Erfinder: Hubschwerlen, Christian, F-68200 Durmenach (FR); Richter, Hans, D-79639 Grenzach-Wyhlen (DE); Specklin, Jean-Luc, F-68680 Kembs-Loenchle (FR)
(74) Vertreter: Poppe, Regina

(56) Entgegenhaltungen:
- EP-A- 0 236 074
- EP-A- 0 272 016
- US-A- 4 356 174
- TETRAHEDRON, Bd.43, Nr.5, 1987 Seiten 1003 - 1012 J.E.BALDWIN 'Functionalisation of the 3-beta-methyl group of penicillin'
- THE JOURNAL OF ANTIBIOTICS, Bd.XLII, Nr.1, Januar 1989 Seiten 159 - 162 S. NISHIMURA ET AL 'Synthesis and beta-lactamase inhibitory activity of 7-alpha-hydroxyethyl cephem derivatives'
- TETRAHEDRON, Bd.46, Nr.17, 1990 Seiten 6145 - 6154 J.E.BALDWIN ET AL 'Penicillin biosynthesis'

## Beschreibung

Die vorliegende Erfindung betrifft Penam-Derivate, im speziellen betrifft sie 3β-Alkenyl-penam-Derivate der allgemeinen Formel 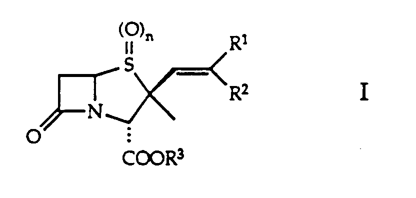 worin
eines von R¹ und R² -COR⁴, -CN, -CH₂R⁵, Halogen, -CH=CHR⁶ oder Q und das andere Wasserstoff oder niederes Alkyl oder beide zusammen einen γ-Lactamring,
- R³: Wasserstoff, niederes Alkyl, Aryl-alkyl, Allyl oder einen in vivo abspaltbaren Rest,
- R⁴: Wasserstoff, niederes Alkyl, niederes Alkoxy, Benzyloxy, Amino, niederes Alkylamino oder niederes Alkyl-niederes Alkoxyamino,
- R⁵: Hydroxy, -OCONHR⁷, -OCONH₂ oder einen fünf- oder sechsgliedrigen, N,S und/oder O enthaltenden hetero-aromatischen, über ein Stickstoffatom verknüpften Ring,
- R⁶: -CN oder -CHO,
- R⁷: -COCH₂Cl,
- Q: einen fünf- oder sechsgliedrigen, N,S und/oder O enthaltenden hetero-aromatischen Ring und
- n: 0, 1 oder 2 bedeuten,
sowie die pharmazeutisch verträglichen Salze dieser Verbindungen.

Diese Verbindungen sind neu und zeichnen sich durch therapeutisch wertvolle Eigenschaften aus. Insbesondere besitzen sie ausgeprägte β-Lactamase hemmende Wirkungen und sind somit nützlich bei der Bekämpfung von β-Lactamase bildenden Krankheitserregern in Kombination mit β-Lactam-Antibiotika, wie den Penicillinen und Cephalosporinen.

Weiterhin weisen sie eine antibakterielle Eigenaktivität gegen einzelne Bakterienstämme, wie z. B. Acinetobacter spp., auf.

Der Ausdruck "niederes Alkyl" für sich allein genommen oder in Kombination, wie "niederes Alkoxy" oder "niederes Alkylamino" bedeutet geradkettige oder verzweigte gesättigte Kohlenwasserstoffreste mit höchstens 7, vorzugsweise höchstens 4 Kohlenstoffatomen, wie Methyl, Ethyl, Isopropyl oder t-Butyl.

Der Ausdruck "Aryl-alkyl" umfasst Benzyl, Benzhydryl, p-Methoxybenzyl oder p-Nitrobenzyl.

Der Ausdruck "Halogen" umfasst Fluor, Chlor, Brom oder Jod.

Der Ausdruck " fünf- oder sechsgliedriger, N,S und/oder O enthaltener hetero-aromatischer Ring" bedeutet vorzugsweise 2-Pyridyl, 1-Methylpyridin-2-ylio, 1,3-Thiazol-2-yl, 1,2,4-Oxadiazol-3-yl und dergleichen.

Ein in vivo abspaltbarer Rest bedeutet einen für eine oral verfügbare Anwendung geeigneten Rest, der vorzugsweise eine Estergruppe enthält, wie beispielsweise -CH₂OOCC(CH₃)₃, 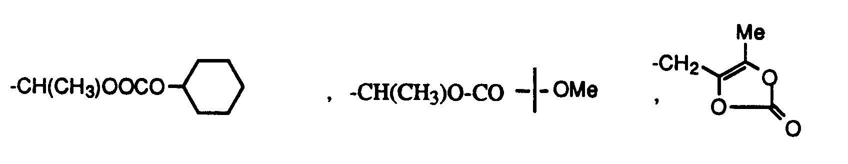
oder -CH(CH₃)OCO-CH₃.

Gegenstand der vorliegenden Erfindung sind 3β-Alkenyl-penam-Derivate der obigen allgemeinen Formel I und pharmazeutisch verträgliche Salze davon als solche und als pharmazeutische Wirkstoffe, die Herstellung dieser Verbindungen, ferner Arzneimittel, enthaltend eine Verbindung der allgemeinen Formel I oder pharmazeutisch verträgliche Salze und gegebenenfalls zusätzlich ein β-Lactam-Antibiotikum und die Herstellung solcher Arzneimittel, sowie die Verwendung von Verbindungen der allgemeinen Formel I und von pharmazeutisch verträglichen Salzen davon bei der Bekämpfung bzw. Verhütung von Krankheiten, insbesondere von bakteriellen Infektionen bzw. zur Herstellung entsprechender Arzneimittel.

Weiterhin sind Gegenstand der Erfindung Verbindungen der allgemeinen Formel 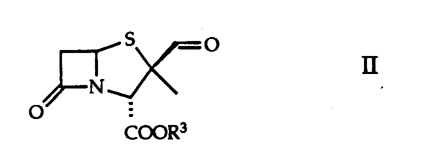
worin R³ die obige Bedeutung hat.

Diese Verbindungen der Formel II sind wichtige Zwischenprodukte für die Herstellung der Verbindungen der allgemeinen Formel I.

Besonders bevorzugt sind solche Verbindungen der allgemeinen Formel I, worin R¹ = CN, Halogen oder COR⁴ und R⁴ = Amino, Methyl, niederes Alkoxy oder Benzyloxy bedeuten und R² = Wasserstoff ist, z.B. die folgenden Verbindungen:
(E/Z)-(2S,3S,5R)-3-(2-Cyanoethenyl)-3-methyl-7-oxo-4-thia-1-aza-bicyclo[3.2.0]heptan-2-carbonsäure-benzhydrylester,
(E)-(2S,3S,5R)-3-(2-Cyanoethenyl)-3-methyl-4,4,7-trioxo-4-thia-1-aza-bicyclo[3.2.0]heptan-2-carbonsäure-benzhydrylester,
(Z)-(2S,3S,5R)-3-(2-Cyanoethenyl)-3-methyl-4,4,7-trioxo-4-thia-1-aza-bicyclo[3.2.0]heptan-2-carbonsäure-benzhydrylester,
(E)-(2S,3S,5R)-3-(2-Cyanoethenyl)-3-methyl-4,4,7-trioxo-4-thia-1-aza-bicyclo[3.2.0]heptan-2-carbonsäure-Natriumsalz,
(Z)-(2S,3S,5R)-3-(2-Cyanoethenyl)-3-methyl-4,4,7-trioxo-4-thia-1-aza-bicyclo[3.2.0]heptan-2-carbonsäure-Natriumsalz,
(Z)-(2S,3S,5R)-3-(2-Chlor-vinyl)-3-methyl-4,4,7-trioxo-4-thia-1-aza-bicyclo[3.2.0]heptan-2-carbonsäure-Natriumsalz,
(E)-(2S,3S,5R)-3-Methyl-3-(3-oxo-but-1-enyl)-4,4,7-trioxo-4-thia-1-aza-bicyclo[3.2.0]heptan-2-carbonsäure-Natriumsalz.

Weiterhin gehören zu den besonders bevorzugten Verbindungen:
(E/Z)-(2S,3S,5R)-3-(2-Carbamoyl-vinyl)-3-methyl-7-oxo-4-thia-1-aza-bicyclo[3.2.0]heptan-2-carbonsäure-benzhydrylester,
(E)-(2S,3S,5R)-3-(2-Carbamoyl-vinyl)-3-methyl-4,4,7-trioxo-4-thia-1-aza-bicyclo[3.2.0]heptan-2-carbonsäure-benzhydrylester,
(Z)-(2S,3S,5R)-3-(2-Carbamoyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Carbamoyl-vinyl)-3-methyl-4,4,7-trioxo-4-thia-1-aza-bicyclo[3.2.0]heptan-2-carbonsäure-Natriumsalz,
(Z)-(2S,3S,5R)-3-(2-Carbamoyl-vinyl)-3-methyl-4,4,7-trioxo-4-thia-1-aza-bicyclo[3.2.0]heptan-2-carbonsäure-Natriumsalz,
(E)-(2S,3S,5R)-3-(2-Ethoxycarbonyl-vinyl)-3-methyl-7-oxo-4-thia-1-aza-bicyclo[3.2.0]heptan-2-carbonsäure-benzhydrylester,
(E)-(2S,3S,5R)-3-(2-Ethoxycarbonyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Ethoxycarbonyl-vinyl)-3-methyl-4,4,7-trioxo-4-thia-1-aza-bicyclo[3.2.0]heptan-2-carbonsäure-Natriumsalz,
(E)-(2S,3S,5R)-3-(2-Benzyloxycarbonyl-vinyl)-3-methyl-7-oxo-4-thia-1-aza-bicyclo[3.2.0]heptan-2-carbonsäure-benzhydrylester,
(E)-(2S,3S,5R)-3-(2-Benzyloxycarbonyl-vinyl)-3-methyl-4,4,7-trioxo-4-
thia-1-aza-bicyclo[3.2.0]heptan-2-carbonsäure-benzhydrylester,
(E)-(2S,3S,5R)-3-(2-Benzyloxycarbonyl-vinyl)-3-methyl-4,4,7-trioxo-4-thia-1-aza-bicyclo[3.2.0]heptan-2-carbonsäure-Natriumsalz.

Die 3β-Alkenyl-penam-Derivate der allgemeinen Formel I sowie deren pharmazeutisch verträglichen Salze können erfindungsgemäss hergestellt werden, indem man
a) eine Verbindung der allgemeinen Formel 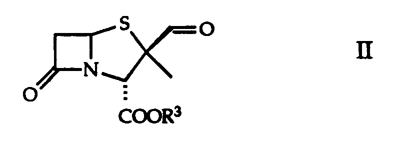 worin R³ die oben angegebene Bedeutung hat,
   mit einer Verbindung der allgemeinen Formel 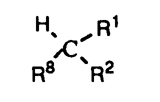 worin R¹ und R² die oben angegebene Bedeutung haben und R⁸ = P^{⊕}(Aryl)₃, PO(OAlkyl)₂, Si(Alkyl)₃ oder Halogen bedeutet,
   in Gegenwart eines Aktivierungsmittels umsetzt, oder
b) eine Verbindung der allgemeinen Formel I, worin n 0 bedeutet, oxydiert oder
c) eine Verbindung der allgemeinen Formel I, worin R³ von Wasserstoff verschieden ist, in die entsprechende freie Säure überführt und
d) erwünschtenfalls eine saure Verbindung der Formel I in ein pharmazeutisch annehmbares Salz überführt.

Zwei bekannte Methoden zur Durchführung von Verfahrensvariante a) zeigt das Schema 1 (Wittig- bzw. Horner-Reaktion).

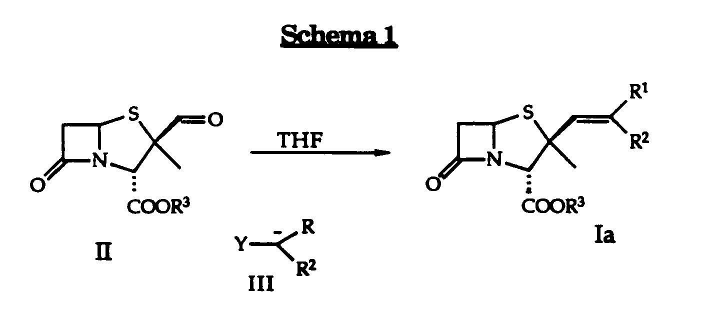

Die Substituenten R¹, R² und R³ entsprechen den obigen Bedeutungen.

Der Aldehyd der Formel II wird durch direkte Umsetzung mit einem Alkyliden-triphenylphosphoran (III; Y = P^{⊕}(Aryl)₃, Wittig-Reaktion) bzw. mit einem Alkylphosphonat (III; Y = (AlkylO)₂PO, Horner-Reaktion) in Gegenwart eines Aktivierungsmittels, beispielsweise einer Base wie Triethylamin, Natriummethylat, Lithiumdiisopropylamin oder DBU (1,5-Diaza-bicyclo[4.3.0]non-5-en) in die Alkenyl-substituierten Pename der Formel Ia überführt. Als Lösungsmittel eignen sich z.B. Tetrahydrofuran, Toluol, Dichlormethan, Acetonitril oder Benzol. Die Reaktionstemperatur kann, je nach eingesetztem Lösungsmittel, zwischen -30° und 80°C variieren.

Wahlweise können die Verbindungen der Formel I auch durch Umsetzung von entsprechend substituierten Triphenylphosphoniumhalogeniden mit 1,2-Butylenoxid als Base und Lösungsmittel dargestellt werden. Die bevorzugte Reaktionstemperatur liegt dabei zwischen 50 und 70°C. Man erhält Verbindungen der Formel I, worin n 0 bedeutet.

Weitere Möglichkeiten zur Durchführung von Verfahrensvariante a) stellen die Peterson-Olefinierung sowie die Reformatsky-Synthese dar. Bei der Peterson-Olefinierung werden Lithiumsalze von Tetraalkylsilanen mit Verbindungen der Formel II umgesetzt. Das Schema 2 zeigt die Umsetzung mit einem entsprechend substituierten Lithium-trimethylsilylmethyl-Derivat der Formel IV, wobei R¹ und R³ die oben angegebenen Bedeutungen haben.

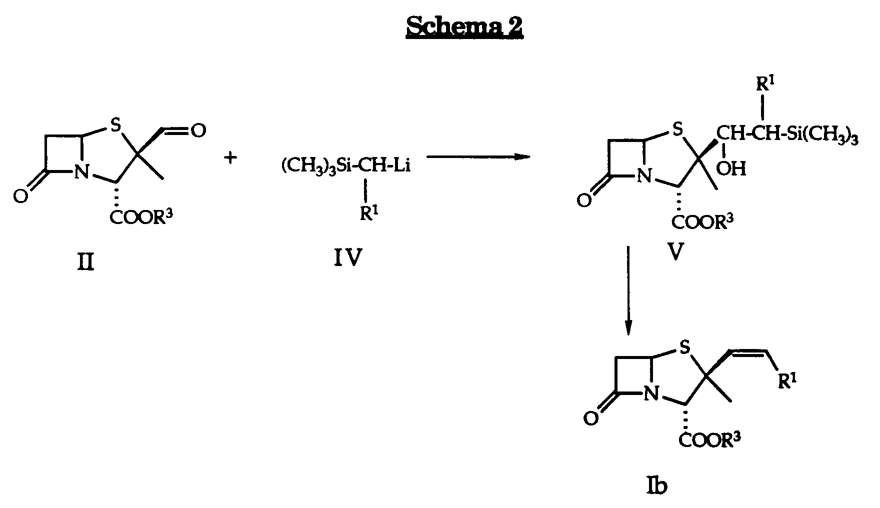

Die 3β-Alkenyl-penam-Derivate der Formel Ib entstehen aus den Verbindungen der Formel V durch spontane Dehydratisierung. Man erhält Verbindungen der Formel I, worin n 0 und R² Wasserstoff bedeuten. Bei der Reformatsky-Synthese werden Verbindungen der Formel II mit Organo-Zink-Derivaten von α-Halogenestern zu den entsprechenden β-Hydroxylestern der Formel VI kondensiert, wobei durch Wasserabspaltung Verbindungen der Formel I entstehen, worin n 0, R¹ COR⁴, R⁴ niederes Alkyl und R² Wasserstoff bedeuten, entsprechend Schema 3.

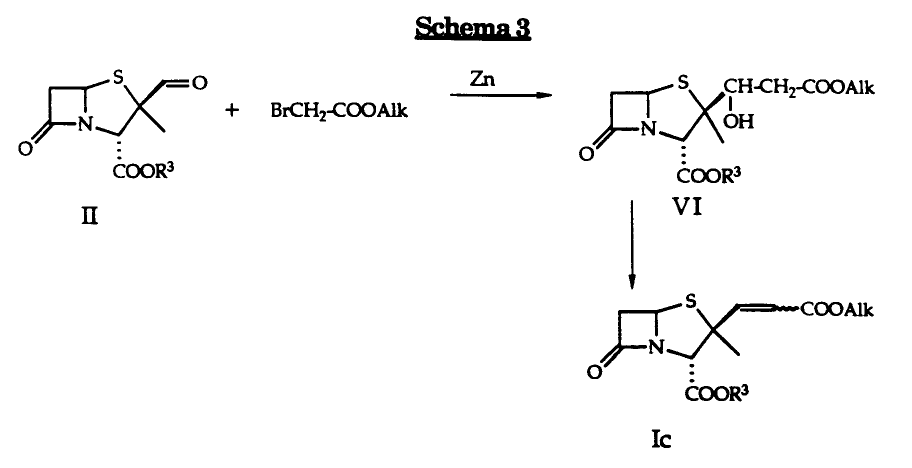

R³ hat die oben angegebene Bedeutung.

Die Oxydation der Sulfid- zur Sulfoxid- oder Sulfongruppe erfolgt nach an sich bekannten Methoden. Besonders geeignet erweist sich die Reaktion der Verbindungen der allgemeinen Formel I, worin n 0 bedeutet, in einem Zweiphasensystem mit Rutheniumtetroxid oder mit einer wässrigen Kaliumpermanganatlösung sowie mit einer Wasserstoffperoxidlösung.

Zur Darstellung der Penam-Sulfone können auch andere Oxydationsreagentien wie Na₂WO₄ oder Persäure herangezogen werden. Als Lösungsmittel ist Dichlormethan besonders geeignet.

Die Verbindung der allgemeinen Formel I, worin n 0 bedeutet, wird zweckmässigerweise in Dichlormethan gelöst und zu einer wässrigen Suspension, bestehend aus Natrium(meta)perjodat, Natriumbicarbonat sowie Ruthenium(IV)oxid gegeben. Nach beendeter Reaktion wird die organische Phase abgetrennt, gereinigt und getrocknet.

Ob bei dieser Oxydation ein Sulfoxid oder ein Sulfon entsteht, hängt von Natur und/oder Menge des Oxydationsmittels ab.

Die Ueberführung einer Verbindung der Formel I, worin R³ von Wasserstoff verschieden ist, in eine freie Säure und die Ueberführung einer freien Säure in ein pharmazeutisch annehmbares Salz kann nach an sich bekannten Methoden erfolgen, unter Umständen in einem einzigen Arbeitsgang.

Estergruppen, z.B. Benzyl, p-Nitrobenzyl, Benzhydryl, p-Methoxybenzyl oder Allyl können wie folgt abgespalten werden:
Benzyl und p-Nitrobenzyl durch Hydrierung über Palladiumkohle bei etwa 0°C bis 80°C in einem organischen Lösungsmittel wie Aethylacetat, Methanol oder Wasser oder durch Hydrolyse in Gegenwart von Natriumsulfid bei ca. 0°C bis Zimmertemperatur in einem Lösungsmittel, wie z.B. DMF. Allyl durch Palladium-(0)-katalysierte Transallylierung in Gegenwart eines Natrium- oder Kaliumsalzes der 2-Aethylcapronsäure, Benzhydryl mit m-Kresol bei ca 50°C innerhalb von 4-5 Stunden.

Die als Ausgangsstoffe verwendeten Verbindungen der allgemeinen Formel II können in Analogie zu einem in Tetrahedron 43, Nr. 5, S. 1003-1012, (1987) beschriebenen Verfahren aus entsprechenden Alkoholen gewonnen werden.

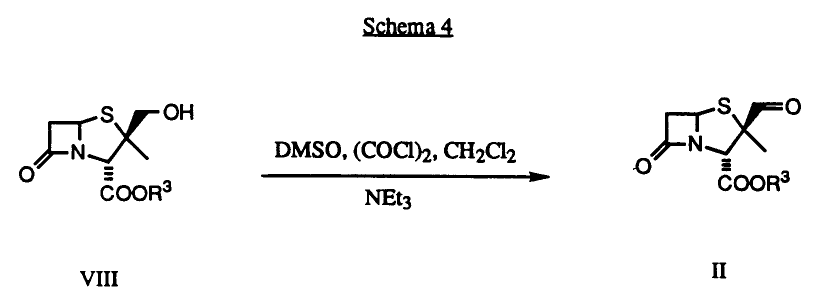

Dabei hat R³ die oben angegebene Bedeutung.

Von Vorteil erweist sich die Umsetzung mit Oxalylchlorid in Dichlormethan, wobei man zweckmässigerweise wie folgt vorgeht: Dichlormethan und das Oxydationsmittel werden auf ca. -60°C abgekühlt, mit DMSO und einer Verbindung der Formel VIII versetzt und nach ca. 3-stündigem Reaktionsverlauf und Zugabe von Triethylamin wird das Kältebad entfernt. Der so entstandene Aldehyd der Formel II kann mit üblichen Methoden gereinigt werden.

Analog erhält man Verbindungen der allgemeinen Formel II durch Oxydation mit PCC (Pyridin-Chlorchromat), Dess-Martin-Reagenz, MnO₂ u.a.

Wie bereits eingangs erwähnt, zeigen die erfindungsgemässen Verbindungen der allgemeinen Formel I und pharmazeutisch verträgliche Salze davon mit Basen ausgeprägte β-Lactamase hemmende Aktivitäten gegen β-Lactamasen aus verschiedenen Bakterienstämmen. Diese therapeutisch wertvollen Eigenschaften können, wie nachstehend erläutert, an isolierten β-Lactamasen in vitro bestimmt werden.

### A. Isolierung der β-Lactamasen

Verschiedene β-Lactamasen können aus penicillin- bzw. cephalosporin-resistenten Bakterienstämmen, wie Klebsiella pneumoniae NCTC 418, Proteus vulgaris 1028, Bacillus licheniformis 749/C, Escherichia coli SN01 und Citrobacter freundii 1203 isoliert werden. Hierzu werden die entsprechenden Stämme in Tryptic Soy Broth (Difco) kultiviert und durch Zentrifugation in der späten logarithmischen Wachstumsphase geerntet (wenn nötig gibt man dem Medium gegen Ende der log-Phase 50-100 mg/l Ampicillin hinzu, um die β-Lactamase zu induzieren). Die so erhaltene Bakterienmasse wird mit 20 mM Tris-HCl-Puffer (pH 7,0) versetzt; unter Kühlung werden die Zellen mit French Press aufgebrochen. Man zentrifugiert (20'000 U/min.) während 20-30 Minuten und erhält einen klaren Rohextrakt. Die Reinigung der Proteine erfolgt nach der Methode von Cartwright, S.J. & Waley, S.G. [Biochem. J. 221, 505-512 (1980)] und, für B. licheniformis, Ellerby, L.M. et al. [Biochemistry 29, 5797-5806 (1990)].

### B. Bestimmung der β-Lactamase-Aktivität

Die Bestimmung der Aktivität der isolierten β-Lactamasen kann nach der Methode von O'-Callaghan, C.H. et al. [Antimicr. Ag. Chemother 1, 283-288 (1972)] mit dem chromogenen Cephalosporin Nitrocefin (87/312 von Glaxo) durchgeführt werden. Der benötigte Versuchsansatz enthält pro ml Wasser: 50 mM Phosphatpuffer (pH 7,0), 0,1 mM Nitrocefin und genügend Enzym (β-Lactamase) um eine ΔA/min. von ca. 0,1 zu erreichen. Die Spaltung des Substrates, die mit einer Farbänderung verbunden ist, erfolgt bei 37°C und wird bei 482 nm mit einem Spektralphotometer quantitativ verfolgt.

### C. Bestimmung der β-Lactamase hemmenden Wirkung der Verbindungen der allgemeinen Formel I

Die oben beschriebene Spaltung des chromogenen Substrates durch β-Lactamasen (Versuch B.) kann durch Zugabe von Verbindungen der allgemeinen Formel I (Inhibitoren) gehemmt werden. Da es sich zeigte, dass die Inhibitoren die β-Lactamase in einer zeitabhängigen Reaktion irreversibel inaktivieren, wird die Reaktion (Spaltung des Substrates), jeweils nach einer Vorinkubationszeit von β-Lactamase mit Inhibitor von 15 Minuten, durch Zugabe des Substrates gestartet. Als Mass für die Affinität des jeweils getesteten Inhibitors zur β-Lactamase, die ein Mass für die Stärke des Inhibitors darstellt, dient diejenige Konzentration, welche die unter obigen Versuchsbedingungen (Versuch B.) in Abwesenheit eines Inhibitors erfolgende Spaltung des Substrates (Nitrocefin) zu 50% hemmt (IC₅₀ in nM). Zur Bestimmung der IC₅₀ wurden 4 bis 6 Versuche mit verschiedenen Konzentrationen an Inhibitor durchgeführt. Die Ermittlung der IC₅₀ erfolgte graphisch.

Die in obigem Versuch (Versuch C) erhaltenen Resultate sind in den nachfolgenden Tabellen 1 bis 3 dargestellt.

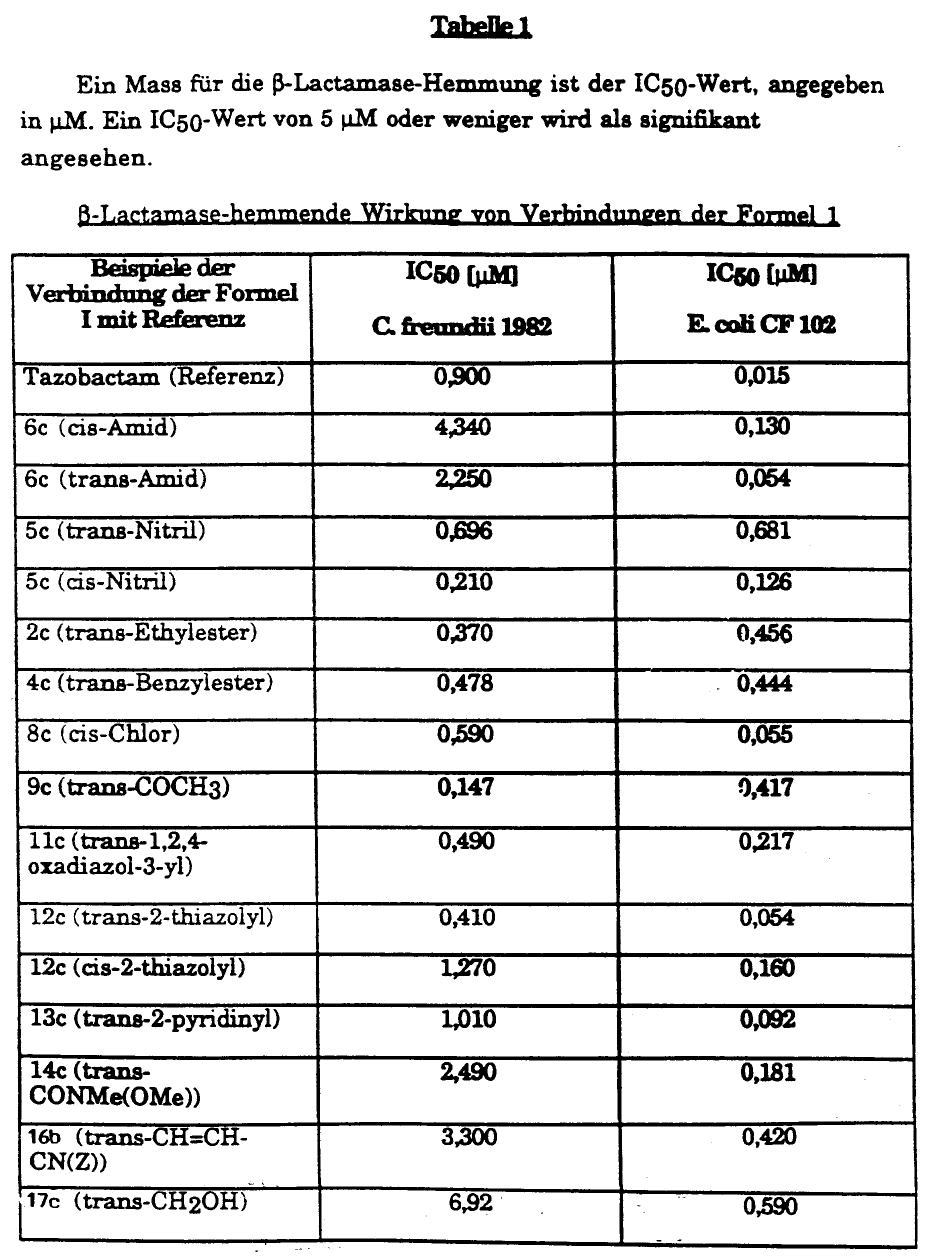

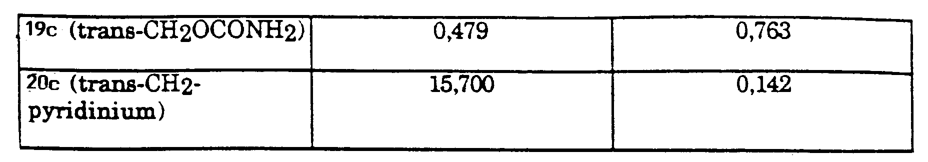

**Tabelle 2**

| β-Lactamase-hemmende Wirkung durch Kombination von Verbindungen der Formel I mit Ceftriaxon (1 Teil Ceftriaxon + 4 Teile Hemmer) | | |
|---|---|---|
| **Beispiele der Verbindung der Formel I mit Referenz** | **MHK [µg/ml] C. freundii 1982** | **MHK [µg/ml] E. coli CF 102** |
| Ceftriaxon | 128 | 8 |
| Ceftriaxon + Tazobactam (Referenz) | 8 | 0,25 |
| Ceftriaxon +6c (cis-Amid) | 4 | 0,12 |
| Ceftriaxon + 6c (trans-Amid) | NA | 0,12 |
| Ceftriaxon + 5c (trans-Nitril) | 2 | 0,25 |
| Ceftriaxon + 5c (cis-Nitril) | 1 | 0,25 |
| Ceftriaxon + 2c (trans-Ethylester) | 4 | 0,25 |
| Ceftriaxon + 4c (trans-Benzylester) | 16 | 2,00 |
| Ceftriaxon + 8c (cis-Chlor) | 4 | 0,25 |
| Ceftriaxon + 9c (trans-COCH₃) | 1 | 0,25 |
| Ceftriaxon + 11c (trans-1,2,4-oxadiazol-3-yl) | 4 | 0,5 |
| Ceftriaxon + 12c (trans-2-thiazolyl) | 8 | 1 |
| Ceftriaxon + 12c (cis-2-thiazolyl) | 16 | 0,5 |
| Ceftriaxon + 13c (trans-2-pyridinyl) | 8 | 1 |
| Ceftriaxon + 14c (trans-CONMe(OMe)) | 8 | 0,5 |
| Ceftriaxon + 16b (trans-CH=CH-CN(Z)) | 8 | 0,5 |
| Ceftriaxon + 17c (trans-CH₂OH) | 8 | 0,5 |
| Ceftriaxon + 19c (trans-CH₂OCONH₂) | 2 | 1 |
| Ceftriaxon + 20c (trans-CH₂-Pyridinium) | 8 | 0,25 |
| MHK bedeutet minimale Hemmkonzentration. | | |

**Tabelle 3**

| In vitro-Aktivität von Kombinationen des Penam-Sulfon-Derivates 5c mit ausgewählten Antibiotika gegen β-Lactamase überproduzierende Stämme (konstante Konzentration des Hemmers: 4 mg/l) | | | | |
|---|---|---|---|---|
| | **MHK E. cloacae P99** | **MHK C.freundii 1982** | **MHK Ps.aeruginosa 18SH** | **MHK E.coli CF-102** |
| Ceftriaxon | >64 | 64 | >64 | 16 |
| Ceftriaxon + 5c (cis-Nitril) | 4 | 2 | 16 | ≤0,12 |
| Ceftriaxon + Tazobactam | 64 | 32 | >64 | ≤0,12 |
| Ceftazidim | >64 | >64 | 32 | 32,00 |
| Ceftazidim + 5c (cis-Nitril) | 4 | 8 | 2 | 0,5 |
| Ceftazidim + Tazobactam | 64 | 64 | 8 | 0,5 |
| Piperacillin | >128 | >128 | >64 | >64 |
| Piperacillin + 5c (cis-Nitril) | 8 | 8 | 16 | 4 |
| Piperacillin + Tazobactam | 128 | 64 | 64 | 4 |
| Apalcillin | >128 | >128 | 64 | >64 |
| Apalcillin + 5c (cis-Nitril) | 16 | 8 | 4 | 2 |
| Apalcillin + Tazobactam | >128 | 64 | NA | 2 |

Die erfindungsgemässen Produkte können als Heilmittel, z.B. in Form pharmazeutischer Präparate Verwendung finden, welche sie oder ihre Salze in Mischung mit einem für die parenterale oder enterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln, oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anästhetika oder Puffer. Die Verbindungen der Formel I und ihre Salze kommen vorzugsweise für die parenterale Applikation in Betracht und werden zu diesem Zweck bevorzugt als Lyophilisate oder Trockenpulver zur Verdünnung mit üblichen Agenzien wie Wasser oder isotonischer Kochsalzlösung, zubereitet.

Wie eingangs erwähnt, kann man die Verbindungen der allgemeinen Formel I und ihre pharmazeutisch verträglichen Salze erfindungsgemäss bei der Bekämpfung bzw. Verhütung von Krankheiten verwenden, insbesondere bei der Bekämpfung von β-Lactamase bildenden Krankheitserregern in Kombination mit β-Lactam-Antibiotika, d.h. Antibiotika, welche einen β-Lactamring enthalten, beispielsweise Penicilline, wie Piperacillin, Mezlocillin, Azlocillin, Apalcillin, Benzylpenicillin, Phenoxymethylpenicillin, Carbenicillin, Methicillin, Propiocillin, Ticarcillin, Ampicillin, Amoxicillin oder Mecillinam, und Cephalosporine, wie Ceftriaxon, Ceftazidim, Cefetamet, Cefetamet Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, Cefpirom oder Cefepim, sowie Peneme und Carbapeneme wie Imipenem oder Meropenem. Dabei können die Verbindungen der allgemeinen Formel I oder pharmazeutisch verträgliche Salze davon mit Basen vor, gleichzeitig mit oder nach der Verabreichung bzw. Einnahme von β-Lactam-Antibiotika verabreicht werden. Werden die erfindungsgemässen Produkte gleichzeitig mit einem β-Lactam-Antibiotikum verabreicht, so kann dies durch Verabreichung als ad-hoc-Kombination oder in Form einer pharmazeutischen Kombination erfolgen, welche eine Verbindung der allgemeinen Formel I oder ein pharmazeutisch verträgliches Salz davon mit Base und ein β-Lactam-Antibiotikum enthält; derartige pharmazeutische Kombinationen sind ebenfalls Gegenstand der vorliegenden Erfindung.

Die Dosierung der Verbindungen der allgemeinen Formel I und der pharmazeutisch verträglichen Salze davon mit Basen kann innerhalb weiter Grenzen variieren und ist natürlich in jedem einzelnen Fall den individuellen Gegebenheiten und dem zu bekämpfenden β-Lactamase produzierenden Krankheitserreger anzupassen. Im allgemeinen dürfte eine Tagesdosis von etwa 0,1 bis etwa 2,0 g angemessen sein. Das Verhältnis von β-Lactamase-Inhibitor (Verbindung der Formel I oder pharmazeutisch verträgliches Salz davon mit Base) zu β-Lactam-Antibiotikum kann ebenfalls innerhalb weiter Grenzen variieren und ist in jedem einzelnen Fall den individuellen Gegebenheiten anzupassen. Im allgemeinen dürfte ein Verhältnis von etwa 1:20 bis etwa 1:1 angemessen sein.

Wie eingangs erwähnt, sind Arzneimittel enthaltend eine Verbindung der allgemeinen Formel I oder ein pharmazeutisch verträgliches Salze davon ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch verträgliche Salze davon und gegebenenfalls eine oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Insbesondere sind pharmazeutische Kombinationen enthaltend eine Verbindung der allgemeinen Formel I oder ein pharmazeutisch verträgliches Salz davon und ein β-Lactam-Antibiotikum, z.B. ein Penicillin, wie Piperacillin, Mezlocillin, Azlocillin, Apalcillin, Benzylpenicillin, Phenoxymethylpenicillin, Carbenicillin, Methicillin, Propicillin, Ticarcillin, Ampicillin, Amoxicillin oder Mecillinam, oder ein Cephalosporin, wie Ceftriaxon, Ceftazidim, Cefetamet, Cefetamet-Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, Cefpirom, Cefepim sowie Peneme und Carbapeneme wie Imipenem oder Meropenem Gegenstand der vorliegenden Erfindung. Derartige Kombinationen eignen sich zur Bekämpfung von β-Lactamase bildenden Krankheitserregern.

In den nachfolgenden Beispielen soll die vorliegende Erfindung näher erläutert werden, ohne ihren Inhalt jedoch in irgend einer Weise zu beschränken.

### Beispiel 1

### (2S,3R,5R)-3-Formyl-3-methyl-7-oxo-4-thia-1-aza-bicyclo[3.2.0]heptan-2-carbonsäure benzhydrylester

100 ml Dichlormethan werden unter Argon mit 0.84 ml (9.60 mmol) Oxalylchlorid versetzt und auf -60°C abgekühlt. Hierzu werden 0.73 ml (10.25 mmol) DMSO, gefolgt von 2.50 g (6.52 mmol) (2S,3R,5R)-3-Hydroxymethyl-3-methyl-7-oxo-4-thia-1-aza-bicyclo[3.2.0]heptan-2-carbonsäure benzhydrylester gelöst in 100 ml Dichlormethan zugetropft. Man rührt bei gleicher Temperatur 3 Stunden nach und gibt dann 3.2 ml (23.0 mmol) Triethylamin zu. Man entfernt das Kältebad und lässt auf Raumtemperatur erwärmen. Die orange Lösung wird auf 1000 ml 0.2N Salzsäure gegossen und die wässrige Phase noch zweimal mit Dichlormethan extrahiert. Die organischen Phasen werden mit Wasser und gesättigter, wässriger Natriumchloridlösung gewaschen, über Natriumsulfat getrocknet, filtriert und am Rotationsverdampfer eingeengt. Das so erhaltene Produkt (2.50 g, 100%) kann ohne weitere Reinigung weiterverarbeitet oder aus Diethylether kristallisiert werden.
Ausbeute: 2.00 g (80%) weisses Kristallpulver; Smp.: 111.8-112.8°C
IR (KBr): 2720, 1788, 1743, 1718 cm⁻¹
MS: (M-CH₂CO) 339
¹H-NMR (250MHz, CDCl₃): δ[ppm] = 1.26(s,3H), 3.06(dd,1H,J=16.2Hz, 2Hz, 6-H), 3.54(dd,1H,J=16.2Hz,4Hz,6-H), 5.34(s,1H,2-H), 5.42(dd,1H,J=2Hz, 4Hz,5-H), 6.95(s,1H,C**H**Ph₂), 7.30-7.38(m,10H,Ph), 9.20(s,1H).

### Beispiel 2

### (a)(E)-(2S,3S,5R)-3-(2-Ethoxycarbonyl-vinyl)-3-methyl-7-oxo-4-thia-1-aza-bicyclo[3.2.0]heptan-2-carbonsäure benzhydrylester

500 mg (1.30 mmol) (2S,3R,5R)-3-Formyl-3-methyl-7-oxo-4-thia-1-aza-bicyclo[3.2.0]heptan-2-carbonsäure benzhydrylester werden unter Argon in 20 ml THF gelöst und mit 500 mg (1.40 mmol) Ethoxycarbonylmethylentriphenylphosphoran versetzt. Die gelbe Lösung wird 75 Minuten bei 50°C gerührt und anschliessend eingeengt. Der verbleibende Rückstand wird über Kieselgel (0.040-0.063 mm Korngrösse) mit Essigester : Hexan 9:16 als Laufmittel chromatographiert.
Ausbeute: 520 mg (88%) gelb-oranges Harz;
IR (Film): 1783, 1747, 1717, 1650 cm⁻¹
MS: (M-CONH) 406, (M-CHPh₂) 284
¹H-NMR (250MHz, CDCl₃): δ[ppm] = 1.31(t,3H,J=7Hz), 1.34(s,3H), 3.13(dd,1H,J=2Hz, 16Hz,6-H), 3.57(dd,1H,J=4Hz, 16Hz,6-H), 4.22(q,2H,J=7Hz), 4.78(s,1H,2-H), 5.30(dd,1H,J=4.2Hz,5-H), 5.97(d,1H,J=15Hz,=CH), 6.94(s,1H,C**H**Ph₂), 7.07(d,1H,J=15Hz,=CH), 7.30-7.38(m,10H,Ph).

### (b)(E)-(2S,3S,5R)-3-(2-Ethoxycarbonyl-vinyl)-3-methyl-4,4,7-trioxo-4-thia-1-aza-bicyclo[3.2.0]heptan-2-carbonsäure benzhydrylester

250 mg (0.55 mmol) (E)-(2S,3S,5R)-3-(2-Ethoxycarbonyl-vinyl)-3-methyl-7-oxo-4-thia-1-aza-bicyclo[3.2.0]heptan-2-carbonsäure benzhydrylester werden in 10 ml Dichlormethan gelöst und mit 6.5 ml Eisessig versetzt. Zu der gelben Lösung tropft man 260 mg (1.66 mmol) Kaliumpermanganat in 25 ml Wasser. Nach beendeter Zugabe rührt man noch 30 Minuten nach und versetzt dann das braune Reaktionsgemisch solange mit einer 30%igen Wasserstoffperoxidlösung bis sich ein farbloses Zweiphasengemisch bildet. Man trennt die Phasen im Scheidetrichter und extrahiert die Wasserphase noch zweimal mit Dichlormethan. Die organische Phase wird mit Wasser, gesättigter Natriumhydrogencarbonatlösung sowie gesättigter Natriumchloridlösung gewaschen, über Natriumsulfat getrocknet, filtriert und eingeengt. Das verbleibende Oel wird über Kieselgel (Korngrösse 0.040-0.063 mm) mit Essigester : Hexan 9:16 als Laufmittel chromatographiert.
Ausbeute: 170 mg (60%) farbloser Schaum.
IR (KBr): 1802, 1757, 1721, 1653, 1331, 960 cm⁻¹
MS: (M-H) 482.4
¹H-NMR (250MHz, CDCl₃): δ[ppm] = 1.26(s,3H), 1.35(t,3H,J=7Hz), 3.44-3.59(pseudo-m, 2H, 6-H), 4.29(q,2H,J=7Hz), 4.60(dd,1H,J=4Hz, 2Hz,5-H), 4.61(s,1H,2-H), 5.99(d,1H,J=16Hz,=CH), 6.94(s,1H,C**H**Ph₂), 7.07(d,1H,J=16Hz,=CH), 7.23-7.40(m,10H,Ph).

### (c)(E)-(2S,3S,5R)-3-(2-Ethoxycarbonyl-vinyl)-3-methyl-4,4,7-trioxo-4-thia-1-aza-bicyclo[3.2.0]heptan-2-carbonsäure Natriumsalz

170 mg (0.35 mmol) (E)-(2S,3S,5R)-3-(2-Ethoxycarbonyl-vinyl)-3-methyl-4,4,7-trioxo-4-thia-1-aza-bicyclo[3.2.0]heptan-2-carbonsäure benzhydrylester werden unter Argon in 3 ml m-Kresol gelöst und 4.5 Stunden bei 50°C gerührt. Danach wird mit 12.5 ml Isobutylmethylketon versetzt und die gelborange Lösung dreimal mit je 3 ml gesättigter, wässriger Natriumhydrogencarbonatlösung extrahiert. Die wässrige Phase wird zweimal mit je 5 ml Isobutylmethylketon gewaschen, über einen Faltenfilter filtriert und anschliessend mit konzentrierter Salzsäure auf pH=1 gestellt. Man extrahiert mit Essigester, wäscht die organische Phase mit gesättigter, wässriger Natriumchloridlösung, trocknet über Natriumsulfat, filtriert und engt am Rotationsverdampfer ein. Das verbleibende gelbe Harz wird in 0.5 ml Essigester gelöst und mit 108 µl 2-Ethylcapronsäure-Natriumsalz (2N Lösung in Essigester) versetzt. Man engt ein, versetzt den Rückstand mit 1.5 ml Wasser und extrahiert einmal mit n-Hexan. Anschliessend wird die wässrige Phase über polymeres hydrophobes Gel mit Wasser als Elutionsmittel chromatographiert. Die das Produkt enthaltenden Fraktionen werden vereinigt und lyophilisiert.
Ausbeute: 60 mg (50%) farbloses Lyophilisat
IR (KBr): 1782, 1715, 1627, 1396, 1192 cm⁻¹
MS: (M-Na)⁻ 316.1
¹H-NMR (250MHz, D₂O): δ[ppm] = 1.31(t,3H,J=7Hz), 1.66(s,3H), 3.46(dd,1H,J=16Hz, 2Hz,6-H), 3.71(dd,1H,J=16Hz,4Hz,6-H), 4.28(q,2H,J=7Hz), 4.62(s,1H,2-H), 5.15(dd,1H,J=2Hz,4Hz,5-H), 6.35(d,1H,J=16Hz,=CH), 7.10(d,1H,J=16Hz,=CH).

### Beispiel 3

### (a)(E)-(2S,3S,5R)-3-(2-Methoxycarbonyl-vinyl)-3-methyl-7-oxo-4-thia-1-aza-bicyclo[3.2.0]heptan-2-carbonsäure benzhydrylester

Gemaess Beispiel 2 werden 200 mg (0.52 mmol)(2S,3R,5R)-3-Formyl-3-methyl-7-oxo-4-thia-1-aza-bicyclo[3.2.0]heptan-2-carbonsäure benzhydrylester durch Umsetzung mit 193 mg (0.58 mmol) Methoxycaroonylmethylen-triphenylphosphoran in (E)-(2S,3S,5R)-3-(2-Methoxycarbonyl-vinyl)-3-methyl-7-oxo-4-thia-1-aza-bicyclo[3.2.0]heptan-2-carbonsäure benzhydrylester ueberfuehrt.
Ausbeute: 209 mg (91%) farbloser Schaum
IR (Film): 1782, 1745, 1724, 1651 cm⁻¹
MS: (M+NH₄⁺) 455.3

### Beispiel 4

### (a)(E)-(2S,3S,5R)-3-(2-Benzyloxycarbonyl-vinyl)-3-methyl-7-oxo-4-thia-1-aza-bicyclo[3.2.0]heptan-2-carbonsäure benzhydrylester

763 mg (2.0 mmol) (2S,3R,5R)-3-Formyl-3-methyl-7-oxo-4-thia-1-aza-bicyclo[3.2.0]heptan-2-carbonsäure benzhydrylester werden unter Argon in 15 ml THF gelöst, mit 821 mg (2.0 mmol) Benzyloxycarbonylmethylentriphenylphosphoran versetzt und die orange Lösung 2 Stunden bei 50°C gerührt. Man lässt auf Raumtemperatur abkühlen, entfernt das Lösungsmittel am Rotationsverdampfer und chromatographiert das rotbraune Oel über Kieselgel (Korngrösse 0.040-0.063 mm) mit Essigester : Hexan 9:16 als Laufmittel.
Ausbeute: 800 mg (78%) oranger Schaum
IR (Film): 1782, 1746, 1721, 1649, 985 cm⁻¹
MS: (M+H)⁺ 514.3
¹H-NMR (250MHz, CDCl₃): δ[ppm] = 1.33(s,3H), 3.11(dd,1H,J=16Hz, 1.6Hz,6-H), 3.56(dd,1H,J=16Hz, 4.2Hz,6-H), 4.78(s,1H,2-H), 5.20(s,2H), 5.36(dd,1H, J=1.6Hz, 4.2Hz,5-H), 6.01(d,1H,J=15Hz,=CH), 6.93(s,1H,C**H**Ph₂), 7.12(d,1H,J=15Hz, =CH), 7.30-7.43(m,15H,Ph).

### (b)(E)-(2S,3S,5R)-3-(2-Benzyloxycarbonyl-vinyl)-3-methyl-4,4,7-trioxo-4-thia-1-aza-bicyclo[3.2.0]heptan-2-carbonsäure benzhydrylester

Gemäss Beispiel 2 werden 800 mg (1.56 mmol)(E)-(2S,3S,5R)-3-(2-Benzyloxycarbonyl-vinyl)-3-methyl-7-oxo-4-thia-1-aza-bicyclo[3.2.0]heptan-2-carbonsäure benzhydrylester oxidiert.
Ausbeute: 255 mg (30%) farbloser Schaum
IR(KBr): 1802, 1757, 1723, 1650, 1332 cm⁻¹
MS: (M-H)⁻ 544.2
¹H-NMR (250MHz, CDCl₃): δ[ppm] = 1.25(s,3H), 3.47(dd,1H,J=16Hz, 2Hz,6-H), 3.55(dd,1H,J=16Hz, 4Hz,6-H), 4.66(dd,1H,J=2Hz,4Hz,5-H), 4.81(s,1H,2-H), 5.26(s,2H), 6.02(d,1H,J=16Hz,=CH), 6.93(s,1H,C**H**Ph₂), 7.22(d,1H,J=16Hz,=CH), 7.13-7.14(m,15H,Ph).

### (c)(E)-(2S,3S,5R)-3-(2-Benzyloxycarbonyl-vinyl)-3-methyl-4,4,7-trioxo-4-thia-1-aza-bicyclo[3.2.0]heptan-2-carbonsäure Natriumsalz

Gemäss Beispiel 2 werden 250 mg (0.46 mmol) (E)-(2S,3S,5R)-3-(2-Benzyloxycarbonyl-vinyl)-3-methyl-4,4,7-trioxo-4-thia-1-aza-bicyclo[3.2.0]heptan-2-carbonsäure benzhydrylester entschützt.
Ausbeute: 54 mg (30%) beiges Lyophilisat
IR(KBr): 1781, 1719, 1626, 1322, 1189, 979 cm⁻¹
MS: (M-H)⁻ 378.2
¹H-NMR(250MHz, D₂O): δ[ppm] = 1.65(s,3H), 3.45(dd,1H,J=17Hz,2Hz,6-H), 3.71(dd,1H,J=17Hz,4Hz,6-H), 4.63(s,1H,2-H), 5.14(dd,1H,J=4Hz, 2Hz,5-H), 5.29(s,2H), 6.42(d,1H,J=16Hz,=CH), 7.13(d,1H,J=16Hz,=CH), 7.42-7.55(m,5H,Ph).

### Beispiel 5

### (a)(E/Z)-(2S,3S,5R)-3-(2-Cyanoethenyl)-3-methyl-7-oxo-4-thia-1-aza-bicyclo[3.2.0]heptan-2-carbonsäure benzhydrylester

### Methode A:

Man gibt zu 946 mg (3.14 mmol) Cyanomethylen-triphenylphosphoran unter Argon 1.0 g (2.62 mmol) (2S,3R,5R)-3-Formyl-3-methyl-7-oxo-4-thia-1-aza-bicyclo[3.2.0]heptan-2-carbonsäure benzhydrylester und 30 ml Toluol. Die braune Suspension wird 1 Stunde gerührt, eingeengt und chromatographisch über Kieselgel (Korngrösse 0.040-0.063 mm) mit Essigester : n-Hexan 3:1 als Laufmittel gereinigt.
Ausbeute: 1.0 g (94%) Isomerengemisch, E:Z 3:1
Spektroskopische Daten des Gemisches:
IR(Film): 2224, 1781, 1744 cm⁻¹
MS: (M+Na⁺) 427.2
¹H-NMR (250MHz, CDCl₃) (**E-Isomer**): δ[ppm] = 1.32(s,3H), 3.13(dd,1H,J=16Hz, 1.6Hz, 6-H), 3.63(dd,1H,J=16Hz,4.4Hz,6-H), 4.74(s,1H,2-H), 5.41(dd,1H,J=4.4Hz,1.6Hz,5-H), 5.54(d,1H,J=15Hz,=CH), 6.82(d,1H,J=15Hz,=CH), 6.95(s,1H,C**H**Ph₂), 7.30-7.44(m,10H,Ph).
¹H-NMR(250MHz, CDCl₃) (**Z-Isomer**): δ[ppm] = 1.61(s,3H), 3.18(dd,1H,J=16Hz,1.5Hz,6-H), 3.64(dd,1H,J=16Hz,4.6Hz,6-H), 4.86(s,1H,2-H), 5.41(d,1H,J=12Hz,=CH), 5.44(dd,1H,J=1.5Hz, 4.6Hz,5-H), 6.63(d,1H,J=12Hz,=CH), 6.95(s,1H,C**H**Ph₂), 7.30-7.44(m,10H,Ph).

### Methode B:

Eine Suspension von 2.88 g (9.56 mmol) Cyanomethylentriphenylphosphoran in 24 ml Lithiumperchlorat-Loesung (0.4M in Acetonitril) wird unter Argon auf -20°C abgekuehlt. Dazu tropft man eine Loesung von 3.32 g (8.70 mmol) (2S,3R,5R)-3-Formyl-3-methyl-7-oxo-4-thia-1-aza-bicyclo[3.2.0]heptan-2-carbonsäure benzhydrylester in 30 ml Acetonitril, ruehrt 4 Stunden bei gleicher Temperatur nach und entfernt das Loesungsmittel am Rotationsverdampfer. Der verbleibende Rueckstand wird in 100 ml Essigester aufgenommen, dreimal mit Wasser und einmal mit gesaettigter Kochsalzloesung ausgeschuettelt, ueber Magnesiumsulfat getrocknet, filtriert und eingeengt. Das verbleibende Oel wird ueber Kieselgel (Korngrösse 0.040-0.063 mm) mit Methylenchlorid als Laufmittel gereinigt.
Ausbeute: 3.34 g (94%) Isomerengemisch, E:Z 1:4

### (b)(E/Z)-(2S,3S,5R)-3-(2-Cyanoethenyl)-3-methyl-4,4,7-trioxo-4-thia-1-aza-bicyclo[3.2.0]heptan-2-carbonsäure benzhydrylester

(E/Z)-(2S,3S,5R)-3-(2-Cyanoethenyl)-3-methyl-4,4,7-trioxo-4-thia-1-aza-bicyclo[3.2.0]heptan-2-carbonsäure benzhydrylester wird in der gleichen Weise, wie in Beispiel 2 beschrieben, durch Oxidation von 1.02 g (2.51 mmol) (E/Z)-(2S,3S,5R)-3-(2-Cyanoethenyl)-3-methyl-7-oxo-4-thia-1-aza-bicyclo[3.2.0]heptan-2-carbonsäure benzhydrylester, hergestellt. Die beiden Isomere können dabei chromatographisch ueber Kieselgel (Korngroesse 0.040-0.063 mm) mit Essigester: n-Hexan 9:16 als Laufmittel getrennt werden. Als erste Komponente wird das Z-Isomer eluiert.
Ausbeute: **Z**: 76 mg (7%) farbloses Oel
**E**: 253 mg (23%) farbloser Schaum
Spektroskopische Daten **E-Isomer**:
MS: (M+NH₄)⁺ 454,2
IR (KBr): 2228, 1802, 1758, 1334, 1192, 990 cm⁻¹
¹H-NMR (250MHz, CDCl₃): δ[ppm] = 1.24(s,3H), 3.50(dd,1H,J=16Hz,2.2Hz,6-H), 3.60(dd,1H,J=16Hz,4Hz,6-H), 4.65(dd,1H,J=2Hz,4Hz,5-H), 4.75(s,1H,2-H), 5.37(d,1H,J=16Hz,=CH), 6.85(d,1H,J=16Hz,=CH), 6.98(S,1H,C**H**Ph₂), 7.25-7.45(m,10H,Ph).
Spektroskopische Daten **Z-Isomer**:
MS: (M-H)⁻ 435.3
IR (KBr): 2220, 1801, 1757, 1333, 1190 cm⁻¹
1H-NMR (250MHz, CDCl₃): δ[ppm] = 1.67(s, 3H), 3.48(dd, 1H, J=16Hz, 2.4Hz,6-H), 3.64(dd, 1H, J=16Hz, 4.6Hz,6-H), 4.72(dd, 1H, J=2.4Hz, 4.6Hz,5-H), 4.85(s,1H,3-H), 5.90(d,1H,J=12Hz,=CH), 6.41(d,1H,J=12Hz, =CH), 6.97(s,1H,C**H**Ph₂), 7.26-7.44(m,10H,Ph).

### (c)(E)-(2S,3S,5R)-3-(2-Cyanoethenyl)-3-methyl-4,4,7-trioxo-4-thia-1-aza-bicyclo[3.2.0]heptan-2-carbonsäure Natriumsalz

Gemäss Beispiel 2 werden 215 mg (0.5 mmol) (E)-(2S,3S,5R)-3-(2-Cyanoethenyl)-3-methyl-4,4,7-trioxo-4-thia-1-aza-bicyclo[3.2.0]heptan-2-carbonsäure benzhydrylester entschützt.
Ausbeute: 110 mg (82%) weisses Lyophilisat
IR (KBr): 2240, 1782, 1629, 1397, 1141 cm⁻¹
MS: (M-Na)⁻ 269.0
¹H-NMR (250MHz, CDCl₃): δ[ppm] = 1.64(s,3H), 3.47(dd,1H,J=16Hz,2Hz,6-H), 3.72(dd,1H,J=16Hz,4Hz,6-H), 4.62(s,1H,2-H), 5.15(dd,1H,J=2Hz,4Hz,5-H), 6.07(d,1H,J=16Hz,=CH), 7.08(d,1H,J=16Hz,=CH).

### (Z)-(2S,3S,5R)-3-(2-Cyanoethenyl)-3-methyl-4,4,7-trioxo-4-thia-1-aza-bicyclo[3.2.0]heptan-2-carbonsäure Natriumsalz

95 mg (0.2 mmol) (Z)-(2S,3S,5R)-3-(2-Cyanoethenyl)-3-methyl-4,4,7-trioxo-4-thia-1-aza-bicyclo[3.2.0]heptan-2-carbonsaeure benzhydrylester werden in 3 ml m-Kresol geloest und 45 Minuten bei 50°C geruehrt. Das Reaktionsgemisch wird mit 10 ml Isobutylmethylketon und 120 µl 2-Ethylcapronsaeure-Natriumsalz (2N in Essigester, 1.1Aeq.) versetzt. Man extrahiert zweimal mit je 3 ml Wasser, waescht die vereinigten waessrigen Phasen mit 10 ml Isobutylmethylketon und lyophilisiert. Das gelbe Lyophilisat wird in 1.3 ml Wasser geloest und ueber polymeres hydrophobes Gel chromatographiert.
Ausbeute: 50 mg (80%) farbloses Lyophilisat
IR (KBr): 2222, 1782, 1626, 1395, 1323, 1141 cm⁻¹
¹H-NMR(250MHz, D₂O): δ[ppm] = 1.94(s,3H), 3.47(dd,1H,J=16Hz, 1.6Hz,6-H), 3.73(dd, 1H, J=16Hz, 4.4Hz,6-H), 4.80(s,1H,2-H), 5.19(dd, 1H, J=1.6Hz, 4.4Hz,5-H), 6.15(d.1H,J=12Hz,=CH), 6.68(d,1H,J=12Hz,=CH).

### Beispiel 6

### (a)(E/Z)-(2S,3S,5R)-3-(2-Carbamoyl-vinyl)-3-methyl-7-oxo-4-thia-1-aza-bicyclo[3.2.0]heptan-2-carbonsäure benzhydrylester

2.60 g(6.86 mmol)(2S,3R,5R)-3-Formyl-3-methyl-7-oxo-4-thia-1-aza-bicyclo[3.2.0]heptan-2-carbonsäure benzhydrylester und 2.15 g (6.86 mmol) Carbamoylmethylen-triphenylphosphoran werden unter Argon in 60 ml THF suspendiert und 45 Minuten bei 50°C gerührt. Man nutscht vom Unlöslichen ab und engt das Filtrat am Rotationsverdampfer ein. Das braune Oel wird an Kieselgel (Korngrösse 0.040-0.063 mm) mit Essigester : n-Hexan 1:1 als Laufmittel chromatographiert und die beiden Isomeren getrennt. Das Z-Isomer wird dabei vor dem E-Isomer eluiert, beide können aus CH₂Cl₂/n-Hexan als kristalline Feststoffe erhalten werden.
**Z-Isomer**:
Ausbeute: 217 mg (7.5%) farbloser Feststoff
IR (KBr): 3441, 1777, 1743, 1678, 986 cm-1
MS: (M-CHPh₂) 255
Smp.: 188-189°C
1H-NMR (250MHz, CDCl₃): δ[ppm] = 1.58(s,3H), 3.11(dd,1H,J=16Hz, 1.60Hz,6-H), 3.57(dd,1H,J=16Hz, 4.40Hz,6-H), 5.23(s,1H,2-H), 5.36(dd,1H,J=4.40Hz, 1.60Hz,5-H), 5.58(pseudo-d,2H,NH₂), 5.75(d,1H,J=12Hz,=CH), 6.24(d,1H,J=12Hz,=CH), 6.92(s,1H,C**H**Ph₂), 7.29-7.43(m,10H, Ph).
**E-Isomer**:
Ausbeute: 347 mg (12%) kristalliner Feststoff
IR (KBr): 3444, 1775, 1728, 1671 cm⁻¹
MS: (M+H)⁺ 423.4
Smp.: 142°C (Zers.)
¹H-NMR (250MHz, CDCl₃): δ = 1.36(s,3H), 3.11(dd,1H,J=16Hz, 2.0Hz,6-H), 3.56(dd,1H,J=16Hz, 4.2Hz,6-H), 4.83(s,1H,2-H), 5.37(dd,1H,J=2.0Hz, 4.2Hz,5-H), 5.55(s,br,2H,NH2), 5.97(d,1H,J=15Hz,=CH), 6.94(s,1H,C**H**Ph₂), 6.95(d,1H,J=15Hz,=CH), 7.28-7.40(m,10H,Ph).

### (b)(Z)-(2S,3S,5R)-3-(2-Carbamoyl-vinyl)-3-methyl-4,4,7-trioxo-4-thia-1-aza-bicyclo[3.2.0]heptan-2-carbonsäure benzhydrylester

Gemäss Beispiel 2 werden 370 mg (0.875 mmol) (Z)-(2S,3S,5R)-3-(2-Carbamoyl-vinyl)-3-methyl-7-oxo-4-thia-1-aza-bicyclo[3.2.0]heptan-2-carbonsäure benzhydrylester oxidiert. Als Laufmittel wird ein Gemisch von Essigester : n-Hexan 1:1 verwendet.
Ausbeute: 225 mg (57%) farbloser Schaum
IR (KBr): 1799, 1750, 1678, 1326, 1188, 1140 cm⁻¹
MS: (M+H)⁺ 455,2
¹H-NMR (250MHz, CDCl₃): δ[ppm] = 1.50(s,3H), 3.45(dd,1H,J=16Hz, 2Hz,6-H), 3.56(dd,1H,J=16Hz,4Hz,6-H), 4.67(dd,1H,J=4Hz,2Hz,5-H), 5.28(s,1H,2-H), 5.63(s,br,1H,NH₂), 5.84(d,1H,J=13Hz,=CH), 6.69(s,br,1H,NH₂), 6.39(d,1H,J=13Hz,=CH), 6.93(s,1H,C**H**Ph₂), 7.29-7.41(m,10H,Ph).

### (E)-(2S,3S,5R)-3-(2-Carbamoyl-vinyl)-3-methyl-4,4,7-trioxo-4-thia-1-aza-bicyclo[3.2.0]heptan-2-carbonsaeure benzhydrylester

0.64g (1.51 mmol) (E)-(2S,3R,5R)-3-(2-Carbamoyl-vinyl)-3-methyl-7-oxo-4-thia-1-aza-bicyclo[3.2.0]heptan-2-carbonsaeure benzhydrylester werden in 30 ml Methylenchlorid geloest und auf 0°C abgekuehlt. Man tropft 1.86 g (7.55 mmol) meta-Chlorperbenzoesaeure (Gehalt: 70-75%, 5Aeq.) in 30 ml Methylenchlorid zu, entfernt nach beendeter Zugabe das Kaeltebad und ruehrt 6 Stunden bei Raumtemperatur. Die Reaktionsloesung wird mit Natriumsulfitloesung (3%ig in gesaettigter waessriger Natriumbicarbonatloesung) ausgeschuettelt und mit gesaettigter waessriger Natriumchloridloesung gewaschen. Man trocknet ueber Magnesiumsulfat, filtriert, entfernt das Loesungsmittel am Rotationsverdampfer und kristallisiert den verbleibenden Rueckstand mit Methylenchlorid/n-Hexan um.
Ausbeute: 210 mg (30%) farbloser Feststoff
IR (KBr): 1799, 1756, 1686, 1329, 1143, 1191 cm⁻¹
MS: (M+H⁺) 455.3
Smp.: 186-188°C
¹H-NMR(250MHz, CDCl₃): δ[ppm] = 1.30(s,3H), 3.47(dd,1H,J=16Hz, 3.0Hz,6-H), 3.61(dd,1H,J=16Hz, 4.4Hz,6-H), 4.67(dd,1H,J=3.0Hz, 4.4Hz,5-H), 4.82(s,1H,2-H), 5.53(s,br,2H,NH2), 6.03(d,1H,J=16Hz,=CH), 6.98(d,1H,J=16Hz,=CH), 6.94(s,1H,C**H**Ph₂), 7.26-7.40(m,10H,Ph).

### (c)(Z)-(2S,3S,5R)-3-(2-Carbamoyl-vinyl)-3-methyl-4,4,7-trioxo-4-thia-1-aza-bicyclo[3.2.0]heptan-2-carbonsäure Natriumsalz

Gemäss Beispiel 2 werden 135 mg (0.30 mmol) (Z)-(2S,3S,5R)-3-(2-Carbamoyl-vinyl)-3-methyl-4,4,7-trioxo-4-thia-1-aza-bicyclo[3.2.0]heptan-2-carbonsäure benzhydrylester entschützt.
Ausbeute: 67 mg (70%) farbloses Lyophilisat
IR (KBr): 3434, 1781, 1668, 1626, 1397, 1317, 1139 cm⁻¹
MS: (M-Na)⁻ 287.2
¹H-NMR (250MHz, D₂O): δ[ppm] = 1.76(s,3H), 3.44(dd,1H,J=16Hz,1.2Hz,6-H), 3.69(s,1H,J=16Hz,4Hz,6-H), 4.68(s,1H,2-H), 5.10(dd,1H,J=4Hz,1.2Hz,5-H), 5.94(d,1H,J=13Hz,=CH), 6.53(d,1H,J=13Hz,=CH).

### (E)-(2S,3S,5R)-3-(2-Carbamoyl-vinyl)-3-methyl-4,4,7-trioxo-4-thia-1-azabicyclo[3.2.0]heptan-2-carbonsaeure Natriumsalz

Gemaess Beispiel 2 werden 350 mg (0.78 mmol) (E)-(2S,3S,5R)-3-(2-Carbamoyl-vinyl)-3-methyl-4,4,7-trioxo-4-thia-1-aza-bicyclo[3.2.0]heptan-2-carbonsaeure-benzhydrylester entschuetzt.
Ausbeute: 190 mg (80%)
IR (KBr): 1782, 1684, 1622, 1398, 1318, 1140 cm⁻¹
MS: (M-Na)⁻ 287.1
¹H-NMR (250MHz, D₂O): δ[ppm] = 1.66(s,3H), 3.46(dd,1H,J=17Hz, 1.4Hz,6-H), 3.71(dd, 1H, J=17Hz, 4.0Hz,6-H), 4.62(s,1H,2-H), 5.14(dd,J=1.4Hz, 4.0Hz,5-H), 6.46(d,1H,J=16Hz,=CH), 6.88(d,1H,J=16Hz,=CH).

### Beispiel 7

### (a)(E)-(2S,3S,5R)-3-(2-Ethoxycarbonyl-propenyl)-3-methyl-7-oxo-4-thia-1-aza-bicyclo[3.2.0]heptan-2-carbonsäure benzhydrylester

286 mg (0.75 mmol) (2S,3R,5R)-3-Formyl-3-methyl-7-oxo-4-thia-1-aza-bicyclo[3.2.0]heptan-2-carbonsäure benzhydrylester werden unter Argon in 12 ml THF gelöst und mit 271 mg (0.75 mmol) (1-Ethoxycarbonylethyliden)-triphenylphosphoran versetzt. Die gelbe Lösung wird 6 Stunden bei 70°C gerührt und anschliessend eingeengt. Der verbleibende Rückstand wird über Kieselgel (0.040-0.063 mm Korngrösse) mit Essigester: Hexan 9:16 als Laufmittel chromatographiert.
Ausbeute: 157 mg (45%) blassgelber Feststoff
IR (Film): 1788, 1754, 1704, 1642 cm⁻¹
MS: (M+NH₄⁺) 483.4
¹H-NMR (400MHz, CDCl₃): δ[ppm] = 1.32(t,3H,J=7.1Hz),1.38(s,3H), 2.03(d,3H,J=1.5Hz), 3.05(dd,1H,J=1.8Hz, 16Hz,6-H), 3.57(dd,1H,J=4.3Hz, 16Hz,6-H), 4.23(q,2H,J=7.1Hz), 4.89(s,1H,2-H), 5.29(dd,1H,J=4.3, 1.8Hz,5-H), 6.94(s,1H,C**H**Ph₂), 7.20(d,1H,J=1.5Hz,=CH), 7.30-7.36(m,10H,Ph).

### Beispiel 8

### (a)(E,Z)-(2S,3S,5R)-3-(2-Chlor-vinyl)-3-methyl-7-oxo-4-thia-1-aza-bicyclo[3.2.0]heptan-2-carbonsäure benzhydrylester

Die Suspension von 1.37 g (4.40 mmol) Chlormethylen-triphenylphosphoran in 25 ml Diethylether wird auf 0°C gekuehlt und mit 1.60 g (4.20 mmol) (2S,3R,5R)-3-Formyl-3-methyl-7-oxo-4-thia-1-aza-bicyclo[3.2.0]heptan-2-carbonsäure benzhydrylester versetzt. Man entfernt das Kaeltebad und ruehrt bei Raumtemperatur noch 3 Stunden nach. Die orange Suspension wird filtriert und anschliessend eingeengt. Der verbleibende Rückstand wird über Kieselgel (0.040-0.063 mm Korngrösse) mit Essigester : Hexan 1:2 als Laufmittel chromatographiert.
Ausbeute: 1.20 g (69%) gelbes Isomerengemisch, E:Z 7:9
Spektroskopische Daten des Gemisches:
IR (Film): 1782, 1747, 1590, 1496, 1251 cm⁻¹
MS: (M+NH₄⁺) 413

### (b)(E/Z)-(2S,3S,5R)-3-(2-Chlor-vinyl)-3-methyl-4,4,7-trioxo-4-thia-1-aza-bicyclo[3.2.0]heptan-2-carbonsäure benzhydrylester

### Man loest unter Argon 2.98 g (14.05 mmol) Natrium-metaperiodat in 30 ml

Wasser und kuehlt auf 0°C. Man versetzt mit 0.93g (11.17 mmol) Natriumbicarbonat gefolgt von 45 ml Acetonitril sowie 60 ml Methylenchlorid. Zu dem Zweiphasengemisch gibt man 9 mg (0.070 mmol) Rutheniumdioxid, anschliessend die Loesung von 1.20 g (2.90 mmol) (E/Z)-(2S,3S,5R)-3-(2-Chlor-vinyl)-3-methyl-7-oxo-4-thia-1-aza-bicyclo[3.2.0]heptan-2-carbonsäure benzhydrylester. Das Kaeltebad wird entfernt und das Reaktionsgemisch bis zur vollstaendigen Umsetzung (DC-Kontrolle) geruehrt. Anschliessend wird mit 2 g Aktivkohle und 20 ml gesaetttigter Kochsalzloesung versetzt, 5 Minuten nachgeruehrt und der Reaktionsansatz ueber Dicalit abgenutscht. Die Phasen werden im Scheidetrichter getrennt, die waessrige Phase zweimal mit Methylenchlorid extrahiert und die vereinigten organischen Auszuege mit Wasser und gesaettigter Kochsalzloesung gewaschen. Man trocknet ueber Magnesiumsulfat / Bleicherde, filtriert und rotiert ein. Das verbleibende Oel wird ueber Kieselgel (Korngroesse 0.040-0.063 mm) mit Essigester : n-Hexan 1:2 als Laufmittel chromatographiert, dabei werden die beiden Isomere getrennt. Als erste Komponente wird das Z-Isomer eluiert.
Ausbeute: **Z**: 495 mg (38%) farbloser Schaum
**E**: 385 mg (30%) farbloser Schaum
Spektroskopische Daten **E-Isomer**:
MS: (M+NH₄)⁺ 463.4
IR (KBr): 1800, 1756, 1612, 1331, 1143, 700 cm⁻¹
Spektroskopische Daten **Z-Isomer**:
MS: (M+NH₄)⁺ 463.4
IR (KBr): 1800, 1755, 1630, 1330, 1143, 700 cm⁻¹

### (c)(E)-(2S,3S,5R)-3-(2-Chlor-vinyl)-3-methyl-4,4,7-trioxo-4-thia-1-aza-bicyclo[3.2.0]heptan-2-carbonsäure Natriumsalz

385 mg (0.86 mmol)(**E**)-(2S,3S,5R)-3-(2-Chlor-vinyl)-3-methyl-4,4,7-trioxo-4-thia-1-aza-bicyclo[3.2.0]heptan-2-carbonsäure benzhydrylester werden unter Argon in 2.3 ml m-Kresol geloest und 4 Stunden bei 50°C geruehrt. Anschliessend wird mit 15 ml Isobutylmethylketon verduennt, mit 0.50 ml 2-Ethylcapronsaeure-Natriumsalz (2N Loesung in Essigester) versetzt und dreimal mit je 8 ml Wasser extrahiert. Man waescht die vereinigten Wasserphasen noch einmal mit 10 ml Isobutylmethylketon, lyophilisiert und chromatographiert das erhaltene Lyophilisat ueber polymeres hydrophobes Gel mit Wasser als Elutionsmittel.
Ausbeute: 57 mg (32%)
MS: (M-Na)⁻ 278.2
IR (KBr): 1780, 1626, 1564, 1417, 1329, 1145 cm⁻¹

### (Z)-(2S,3S,5R)-3-(2-Chlor-vinyl)-3-methyl-4,4,7-trioxo-4-thia-1-aza-bicyclo[3.2.0]heptan-2-carbonsäure Natriumsalz

Gemäss Beispiel 8 werden 400 mg (0.90 mmol) (Z)-(2S,3S,5R)-3-(2-Chlorvinyl)-3-methyl-4,4,7-trioxo-4-thia-1-aza-bicyclo[3.2.0]heptan-2-carbonsäure benzhydrylester entschützt.
Ausbeute: 176 mg (65%) farbloses Lyophilisat
MS: (M-Na)⁻ 278.2
IR (KBr): 1779, 1626, 1398, 1321, 1142 cm⁻¹

| Elementaranalyse: C₉H₉ClNO₅SNa (301.676) | | | |
|---|---|---|---|
| ber. | C 35.83 | H 3.01 | N 4.64 |
| gef.#) | C 35.72 | H 3.04 | N 4.65 |

| | | | |
|---|---|---|---|
| #) wasserfrei gerechnet mit 5.53 % Wasser | | | |

### Beispiel 9

### (a)(E)-(2S,3S,5R)-3-Methyl-7-oxo-3-(3-oxo-but-1-enyl)-4-thia-1-aza-bicyclo[3.2.0]heptan-2-carbonsäure benzhydrylester

381 mg (1.00 mmol) (2S,3R,5R)-3-Formyl-3-methyl-7-oxo-4-thia-1-aza-bicyclo[3.2.0]heptan-2-carbonsäure benzhydrylester werden unter Argon in 12 ml THF gelöst und mit 350 mg (1.10 mmol) Acetylmethylentriphenylphosphoran versetzt. Man ruehrt 6 Tage bei 50°C und engt anschliessend ein. Der verbleibende Rückstand wird über Kieselgel (0.040-0.063 mm Korngrösse) mit Essigester : Methylenchlorid 95 : 5 als Laufmittel chromatographiert.
Ausbeute: 221 mg (52%) weisses Harz
IR (Film): 1782, 1681, 1627, 1319, 1141, 980 cm⁻¹
MS: (M-Na)⁻ 286.1

### (b)(E)-(2S,3S,5R)-3-Methyl-3-(3-oxo-but-1-enyl)-4,4,7-trioxo-4-thia-1-aza-bicyclo[3.2.0]heptan-2-carbonsäure benzhydrylester

Gemäss Beispiel 8 werden 221 mg (0.49 mmol) (E)-(2S,3S,5R)-3-Methyl-7-oxo-3-(3-oxo-but-1-enyl)-4-thia-1-aza-bicyclo[3.2.0]heptan-2-carbonsäure benzhydrylester oxidiert. Man chromatographiert einmal ueber Kieselgel (Korngroesse 0.040-0.063 mm) mit Essigester : n-Hexan 9:16 als Laufmittel und kristallisiert aus Methylenchlorid/n-Hexan.
Ausbeute: 95 mg (40%) farblose Kristalle
MS: (M+NH₄)⁺ 471.2
IR (KBr): 1798, 1760, 1682, 1627, 1330, 1193, 1142, 976 cm⁻¹
Smp.: 183-184°C

### (c)(E)-(2S,3S,5R)-3-Methyl-3-(3-oxo-but-1-enyl)-4,4,7-trioxo-4-thia-1-aza-bicyclo[3.2.0]heptan-2-carbonsäure Natriumsalz

Gemäss Beispiel 8 werden 250 mg (0.55 mmol) (E)-(2S,3S,5R)-3-Methyl-3-(3-oxo-but-1-enyl)-4,4,7-trioxo-4-thia-1-aza-bicyclo[3.2.0]heptan-2-carbonsäure benzhydrylester entschuetzt.
Ausbeute: 47 mg (27%) farbloses Lyophilisat
MS: (M-Na)⁻ 286.1
IR (KBr): 1782, 1681, 1627, 1394, 1319, 1141, 980 cm⁻¹

### Beispiel 10

### (a)(E)-(2S,3S,5R)-3-(2-Formyl-vinyl)-3-methyl-7-oxo-4-thia-1-aza-bicyclo[3.2.0]heptan-2-carbonsäure benzhydrylester

3.81 g (10.0 mmol) (2S,3R,5R)-3-Formyl-3-methyl-7-oxo-4-thia-1-aza-bicyolo[3.2.0]heptan-2-carbonsäure benzhydrylester werden unter Argon in 40 ml Methylenchlorid gelöst und mit 3.34 g (11.0 mmol) Formylmethylentriphenylphosphoran versetzt. Die gelbe Lösung wird 3 Tage bei Raumtemperatur gerührt und anschliessend eingeengt. Der verbleibende Rückstand wird über Kieselgel (0.040-0.063 mm Korngrösse) mit tert-Butylmethylether : n-Hexan 2:3 als Laufmittel chromatographiert.
Ausbeute: 528 mg (13%) farbloses Harz
IR (Film): 2738, 1781, 1744, 1717, 1690, 1496, 1178, 986 cm⁻¹
MS: (M+MH₄⁺) 425.6

### Beispiel 11

### (a)(E)-(2S,3S,5R)-3-Methyl-3-[2-(1,2,4-oxadiazol-3-yl)-vinyl]-7-oxo-4-thia-1-aza-bicyclo[3.2.0]heptan-2-carbonsäure benzhydrylester

1.14 g (3.0 mmol) (2S,3R,5R)-3-Formyl-3-methyl-7-oxo-4-thia-1-aza-bicyclo[3.2.0]heptan-2-carbonsäure benzhydrylester werden in 15 ml 1,2-Butylenoxid geloest, mit 1.38 g (3.6 mmol) (1,2,4-Oxadiazol-3-yl)methyltriphenylphosphoniumchlorid versetzt und 10 Stunden rueckflussiert. Anschliessend wird filtriert, einrotiert und das verbleibende braune Oel ueber Kieselgel (0.040-0.063 mm Korngrösse) mit Methylenchlorid als Laufmittel chromatographiert.
Ausbeute: 280 mg (21%) farblose Kristalle
MS: (M+NH₄)⁺ 465.3
IR (KBr): 1792, 1753, 1658, 1492, 1200, 991 cm⁻¹

### (b)(E)-(2S,3S,5R)-3-Methyl-3-[2-(1,2,4-oxadiazol-3-yl)-vinyl]-4,4,7-trioxo-4-thia-1-aza-bicyclo[3.2.0]heptan-2-carbonsäure benzhydrylester

Gemäss Beispiel 8 werden 280 mg (0.63 mmol) (E)-(2S,3S,5R)-3-Methyl-3-[2-(1,2,4-oxadiazol-3-yl)-vinyl]-7-oxo-4-thia-1-aza-bicyclo[3.2.0]heptan-2-carbonsäure benzhydrylester oxidiert. Man chromatographiert einmal ueber Kieselgel (Korngroesse 0.040-0.063 mm) mit Essigester: n-Hexan 9:16 als Laufmittel und kristallisiert aus Methylenchlorid/n-Hexan.
Ausbeute: 95 mg (40%) farblose Kristalle
MS: (M-H)⁻ 478.3
IR (KBr): 1805, 1758, 1650, 1334, 1196 cm⁻¹
Smp.: 157°C

| Elementaranalyse: C₂₄H₂₁N₃O₆S(479.507) | | | |
|---|---|---|---|
| ber. | C 60.12 | H 4.41 | N 8.76 |
| gef. | C 59.94 | H 4.26 | N 8.55 |

### (c)(E)-(2S,3S,5R)-3-Methyl-3-[2-(1,2,4-oxadiazol-3-yl)-vinyl]-4,4,7-trioxo-4-thia-1-aza-bicyclo[3.2.0]heptan-2-carbonsäure

355 mg (0.74 mmol) (E)-(2S,3S,5R)-3-Methyl-3-[2-(1,2,4-oxadiazol-3-yl)-vinyl]-4,4,7 -trioxo-4-thia-1-aza-bicyclo[3.2.0]heptan-2-carbonsäure benzhydrylester werden in 3 ml m-Kresol geloest und 4 Stunden bei 50°C geruehrt. Zu der hellbraunen Loesung gibt man 20 ml n-Hexan, nutscht die ausgefallenen Kristalle ab und waescht mit n-Hexan nach. Das Rohkristallisat wird aus Essigester/n-Hexan umkristallisiert.
Ausbeute: 170 mg (73%) farblose Kristalle
MS: (M-H)⁻ 312.2
IR (KBr): 2900(br), 1811, 1714, 1660, 1331, 1194, 980 cm⁻¹
Smp.: 161°C (Zers.)

| Elementaranalyse: C₁₁H₁₁N₃O₆S (313.284) | | | |
|---|---|---|---|
| ber. | C 42.17 | H 3.54 | N 13.41 |
| gef. | C 42.41 | H 3.64 | N 13.18 |

### (c)(E)-(2S,3S,5R)-3-Methyl-3-[2-(1,2,4-oxadiazol-3-yl)-vinyl]-4,4,7-trioxo-4-thia-1-aza-bicyclo[3.2.0]heptan-2-carbonsäure Natriumsalz

Zu der klaren Loesung von 23 mg (0.27 mmol) Natriumhydrogencarbonat in 10 ml Wasser gibt man in einer Portion 85 mg (0.27 mmol) (E)-(2S,3S,5R)-3-Methyl-3-[2-(1,2,4-oxadiazol-3-yl)-vinyl]-4,4,7-trioxo-4-thia-1-aza-bicyclo[3.2.0]heptan-2-carbonsäure, ruehrt ca. 10 Minuten nach, filtriert und lyophilisiert.
Ausbeute: 90 mg (91%) farbloses Lyophilisat
MS: (M-Na)⁻ 312.2
IR (KBr): 1787, 1627, 1395, 1321, 1192, 970 cm⁻¹

| Elementaranalyse: C₁₁H₁₀N₃O₆SNa (335.266) | | | |
|---|---|---|---|
| ber. | C 39.41 | H 3.01 | N 12.53 |
| gef.#) | C 39.86 | H 3.04 | N 12.51 |

| | | | |
|---|---|---|---|
| #) wasserfrei gerechnet mit 5.82% Wasser | | | |

### Beispiel 12

### (a)(E) und (Z)-(2S,3S,5R)-3-Methyl-3-(2-thiazol-2-yl-vinyl)-7-oxo-4-thia-1-aza-bicyclo[3.2.0]heptan-2-carbonsäure benzhydrylester

1.14 g (3.0 mmol) (2S,3R,5R)-3-Formyl-3-methyl-7-oxo-4-thia-1-aza-bicyclo[3.2.0]heptan-2-carbonsäure benzhydrylester werden in 50 ml 1,2-Butylenoxid geloest, mit 1.53 g (3.86 mmol) (Thiazol-2-yl)methyltriphenylphosphoniumchlorid versetzt und 40 Stunden rueckflussiert. Anschliessend wird filtriert, einrotiert und das verbleibende braune Filtrat ueber Kieselgel (0.040-0.063 mm Korngrösse) mit Methylenchlorid : Essigester 95 : 5 als Laufmittel chromatographiert. Die beiden Isomere werden dabei getrennt, als erste Komponente wird das Z-Isomer eluiert.
**Z-Isomer**:
Ausbeute: 240 mg (17%) gelbes Oel
IR (Film): 1778, 1743, 1493, 1197, 986 cm⁻¹
MS: (M+H⁺) 463.5
**E-Isomer**:
Ausbeute: 855 mg (62%) gelbes Harz
IR (KBr): 1779, 1746, 1492, 1292, 988 cm⁻¹
MS: (M+H)⁺ 463.5

### (b)(E)-(2S,3S,5R)-3-Methyl-4,4,7-trioxo-3-(2-thiazol-2-yl-vinyl)-4-thia-1-aza-bicyclo[3.2.0]heptan-2-carbonsäure benzhydrylester

Gemaess Beispiel 8 werden 200 mg (0.43 mmol) (E)-(2S,3S,5R)-3-Methyl-3-(2-thiazol-2-yl-vinyl)-7-oxo-4-thia-1-aza-bicyclo[3.2.0]heptan-2-carbonsäure benzhydrylester oxidiert. Man chromatographiert ueber Kieselgel (Korngroesse 0.040-0.063 mm) mit Methylenchlorid : Essigester 95:5 als Laufmittel.
Ausbeute: 72 mg (33%) farbloser Schaum
MS: (M+H)⁺ 495.4
IR (KBr): 1799, 1756, 1329, 1192, 1142, 963 cm⁻¹

### (Z)-(2S,3S,5R)-3-Methyl-4,4,7-trioxo-3-(2-thiazol-2-yl-vinyl)-4-thia-1-aza-bicyclo[3.2.0]heptan-2-carbonsäure benzhydrylester

Gemaess Beispiel 8 werden 210 mg (0.45 mmol) (Z)-(2S,3S,5R)-3-Methyl-4,4,7-trioxo-3-(2-thiazol-2-yl-vinyl)-4-thia-1-aza-bicyclo[3.2.0]heptan-2-carbonsäure benzhydrylester oxidiert. Man chromatographiert ueber Kieselgel (Korngroesse 0.040-0.063 mm) mit Methylenchlorid : Essigester 95:5 als Laufmittel.
Ausbeute: 72 mg (33%) farbloser Schaum
MS: (M+H)⁺ 495.4
IR (KBr): 1793, 1754, 1490, 1328, 1184, 1142, 963 cm⁻¹

### (E)-(2S,3S,5R)-3-Methyl-4,4,7-trioxo-3-(2-thiazol-2-yl-vinyl)-4-thia-1-aza-bicyclo[3.2.0]heptan-2-carbonsäure

Analog Beispiel 11 werden 200 mg (0.40 mmol) (E)-(2S,3S,5R)-3-Methyl-3-(2-thiazol-2-yl-vinyl)-4,4,7-trioxo-4-thia-1-aza-bicyclo[3.2.0]heptan-2-carbonsäure benzhydrylester entschuetzt.
Ausbeute: 53 mg (40%) farblose Kristalle
MS: (M+H)⁺ 329.4
IR (KBr): 2800(br), 1791, 1740, 1634, 1321, 1142, 965 cm⁻¹

### (E)-(2S,3S,5R)-3-Methyl-4,4,7-trioxo-3-(2-thiazol-2-yl-vinyl)-4-thia-1-aza-bicyclo[3.2.0]heptan-2-carbonsäure Natriumsalz

Analog Beispiel 11 werden 53 mg (0.16 mmol) (E)-(2S,3S,5R)-3-Methyl-4,4,7-trioxo-3-(2-thiazol-2-yl-vinyl)-4-thia-1-aza-bicyclo[3.2.0]heptan-2-carbonsäure in das entsprechende Natriumsalz ueberfuehrt.
Ausbeute: 55 mg (98%) farbloses Lyophilisat
IR (KBr): 1778, 1627, 1392, 1318, 1192, 1141, 955 cm⁻¹

| Elementaranalyse: C₁₂H₁₁N₂O₅S₂Na (350.339) | | | |
|---|---|---|---|
| ber. | C 41.14 | H 3.16 | N 8.00 |
| gef.#) | C 40.73 | H 3.51 | N 7.92 |

| | | | |
|---|---|---|---|
| #) wasserfrei gerechnet mit 5.9% Wasser | | | |

### (Z)-(2S,3S,5R)-3-Methyl-4,4,7-trioxo-3-(2-thiazol-2-yl-vinyl)--4-thia-1-aza-bicyclo[3.2.0]heptan-2-carbonsäure Natriumsalz

Analog Beispiel 8 werden 160 mg (0.32 mmol) (Z)-(2S,3S,5R)-3-Methyl-4,4,7-trioxo-3-(2-thiazol-2-yl-vinyl)-4-thia-1-aza-bicyclo[3.2.0]heptan-2-carbonsäure benzhydrylester in das entsprechende Natriumsalz ueberfuehrt.
Ausbeute: 60 mg (57%) farbloses Lyophilisat
IR (KBr): 1781, 1623, 1398, 1320, 1193, 1140, 950 cm⁻¹
¹H-NMR (250MHz, CDCl₃): δ[ppm] = 1.52(s,3H), 3.42(dd,1H,J=16Hz, 1.2Hz,6-H), 3.58(dd,1H,J=16Hz, 4.1Hz,6-H), 5.04(dd,1H,J=1.2Hz, 4.1Hz,5-H), 5.19(s,1H,2-H), 6.06(d,1H,J=12.5Hz,=CH), 7.14(d,1H,J=12.5Hz,=CH), 7.75(ABSystem,2H,J=3.5Hz, Thiazol-H).

### Beispiel 13

### (a)(E)-(2S,3S,5R)-3-Methyl-3-(2-pyridin-2-yl-vinyl)-7-oxo-4-thia-1-aza-bicyclo[3.2.0] heptan-2-carbonsäure benzhydrylester

Die Suspension von 353 mg (1.0 mmol) (2-Picolyl)methylentriphenylphosphoran und 381 mg (1.00 mmol) (2S,3R,5R)-3-Formyl-3-methyl-7-oxo-4-thia-1-aza-bicyclo[3.2.0]heptan-2-carbonsäure benzhydrylester in 10 ml Diethylether wird unter Argon 2 Stunden bei Raumtemperatur geruehrt. Man versetzt mit 10 ml Methylenchlorid , nutscht vom Unloeslichen ab und engt ein. Der verbleibende Rueckstand wird ueber Kieselgel (Korngroesse 0.040-0.063 mm) mit Methylenchlorid : Essigester 95:5 als Laufmittel chromatographiert.
Ausbeute: 128 mg (28%) farbloser Schaum
MS: (M·) 456
IR (KBr): 1779, 1747, 1634, 1291, 988 cm⁻¹

### (b)(E)-(2S,3S,5R)-3-Methyl-3-(2-pyridin-2-yl-vinyl)-4,4,7-trioxo-4-thia-1-aza-bicyclo[3.2.0]heptan-2-carbonsäure benzhydrylester

Gemaess Beispiel 8 werden 196 mg (0.43 mmol) (E)-(2S,3S,5R)-3-Methyl-3-(2-pyridin-2-yl-vinyl)-7-oxo-4-thia-1-aza-bicyclo[3.2.0]heptan-2-carbonsäure benzhydrylester oxidiert. Man chromatographiert ueber Kieselgel (Korngroesse 0.040-0.063 mm) mit Methylenchlorid: Essigester 95:5 als Laufmittel.
Ausbeute: 130 mg (62%) farbloser Schaum
MS: (M+H)⁺ 489.4
IR (KBr): 1799, 1756, 1580, 1490, 1327, 1184, 1141, 969 cm⁻¹

### (c)(E)-(2S,3S,5R)-3-Methyl-3-(2-pyridin-2-yl-vinyl)-4,4,7-trioxo-4-thia-1-aza-bicyclo[3.2.0]heptan-2-carbonsäure

Analog Beispiel 10 werden 200 mg (0.41 mmol) (E)-(2S,3S,5R)-3-Methyl-3-(2-pyridin-2-yl-vinyl)-4,4,7-trioxo-4-thia-1-aza-bicyclo[3.2.0]heptan-2-carbonsäure benzhydrylester entschuetzt.
Ausbeute: 85 mg (65%) hellbeiges Kristallpulver
MS: (M+H)⁺ 323.3
IR (KBr): 2700(br), 1790, 1720, 1621, 1318, 1140, 978 cm⁻¹

### (E)-(2S,3S,5R)-3-Methyl-3-(2-pyridin-2-yl-vinyl)-4,4,7-trioxo-4-thia-1-aza-bicyclo[3.2.0]heptan-2-carbonsäure Natriumsalz

Analog Beispiel 10 werden 83 mg (0.17 mmol) (E)-(2S,3S,5R)-3-Methyl-3-(2-pyridin-2-yl-vinyl)-4,4,7-trioxo-4-thia-1-aza-bicyclo[3.2.0]heptan-2-carbonsäure in das entsprechende Natriumsalz ueberfuehrt.
Ausbeute: 89 mg (100%) farbloses Lyophilisat
IR (KBr): 1784, 1629, 1587, 1478, 1396, 1320, 1187, 1141, 980 cm⁻¹

| Elementaranalyse: C₁₄H₁₃N₂O₅SNa (344.317) | | | |
|---|---|---|---|
| ber. | C 48.84 | H 3.81 | N 8.14 |
| gef.#) | C 49.18 | H 4.23 | N 8.17 |

| | | | |
|---|---|---|---|
| #) wasserfrei gerechnet mit 6.86% Wasser | | | |

### Beispiel 14

### (a)(E)-(2S,3S,5R)-3-[2-(Methoxy-methyl-carbamoyl)-vinyl]-3-methyl-7-oxo-4-thia-1-aza-bicyclo[3.2.0]heptan-2-carbonsäure benzhydrylester

Die orange Loesung von 1.14 g (3.0 mmol) (2S,3R,5R)-3-Formyl-3-methyl-7-oxo-4-thia-1-aza-bicyclo[3.2.0]heptan-2-carbonsäure benzhydrylester und 1.14 g (3.15 mmol) (N-Methoxymethylaminocarbonylmethylen)triphenylphosphoran wird unter Argon 3 Tage bei Raumtemperatur geruehrt. Man entfernt das Loesungsmittel am Rotationsverdampfer und chromatographiert ueber Kieselgel (Korngroesse 0.040-0.063 mm) mit Essigester : n-Hexan 1:1 als Laufmittel.
Ausbeute: 1.12 g (80%) farbloser Schaum
MS: (M+H)⁺ 467.4
IR (KBr): 1781, 1744, 1662, 1629, 995 cm⁻¹

### (b)(E)-(2S,3S,5R)-3-[2-(Methoxy-methyl-carbamoyl)-vinyl]-3-methyl-4,4,7-trioxo-4-thia-1-aza-bicyclo[3.2.0]heptan-2-carbonsäure benzhydrylester

0.69 g (1.48 mmol) (E)-(2S,3S,5R)-3-[2-(Methoxy-methyl-carbamoyl)-vinyl]-3-methyl-7-oxo-4-thia-1-aza-bicyclo[3.2.0]heptan-2-carbonsäure benzhydrylester werden in Analogie zu Beispiel 8 oxidiert. Man chromatographiert ueber Kieselgel (Korngroesse 0.040-0.063 mm) mit Essigester: n-Hexan 3:2 als Laufmittel.
Ausbeute: 405 mg (55%) farbloser Schaum
MS: (M+H)⁺ 499.4
IR (KBr): 1807, 1753, 1662, 1624, 1318, 1140, 1000 cm⁻¹

### (c)(E)-(2S,3S,5R)-3-[2-(Methoxy-methyl-carbamoyl)-vinyl]-3-methyl-4,4,7-trioxo-4-thia-1-aza-bicyclo[3.2.0]heptan-2-carbonsäure Natriumsalz

Gemaess Beispiel 8 werden 200 mg (0.40 mmol) (E)-(2S,3S,5R)-3-[2-(Methoxy-methyl-carbamoyl)-vinyl]-3-methyl-4,4,7-trioxo-4-thia-1-aza-bicyclo[3.2.0]heptan-2-carbonsäure benzhydrylester entschuetzt.
Ausbeute: 95 mg (67%) farbloses Lyophilisat
MS: (M+H)⁺ 355.3
IR (KBr): 1785, 1650, 1626, 1390, 1322, 1191, 1141, 980 cm⁻¹

| Elementaranalyse: C₁₂H₁₅N₂O₇SNa (354.309) | | | |
|---|---|---|---|
| ber. | C 40.68 | H 4.27 | N 7.91 |
| gef.#) | C 40.37 | H 4.35 | N 7.84 |

| | | | |
|---|---|---|---|
| #) wasserfrei gerechnet mit 2.74 % Wasser | | | |

### Beispiel 15

### (a) (1E,3E)-(2S,3S,5R)-3-(4-Formyl-buta-1,3-dienyl)-3-methyl-7-oxo-4-thia-1-aza-bicyclo[3.2.0]heptan-2-carbonsäure benzhydrylester

(1E,3E)-(2S,3S,5R)-3-(4-Formyl-buta-1,3-dienyl)-3-methyl-7-oxo-4-thia-1-aza-bicyclo[3.2.0]heptan-2-carbonsäure benzhydrylester wird in Analogie zu Beispiel 9 aus 3.81 g (10.0 mmol) (2S,3R,5R)-3-Formyl-3-methyl-7-oxo-4-thia-1-aza-bicyclo[3.2.0]heptan-2-carbonsäure benzhydrylester und 4.55 g (15.0 mmol) Formylmethylen-triphenylphosphoran hergestellt. Man chromatographiert über Kieselgel (0.040-0.063 mm Korngrösse) mit Methylenchlorid : Essigester 98:2 als Laufmittel.
Ausbeute: 347 mg (8%) gelbliches Harz
MS: (M+NH₄)⁺ 451.4
IR (KBr): 1779, 1745, 1680, 1637, 1201, 988 cm⁻¹

### Beispiel 16

### (a) (1E,3E) und (1E,3Z)-(2S,3S,5R)-3-(4-Cyano-buta-1,3-dienyl)-3-methyl-7-oxo-4-thia-1-aza-bicyclo[3.2.0]heptan-2-carbonsaeure benzhydrylester

(1E,3E) und (1E,3Z)-(2S,3S,5R)-3-(4-Cyano-buta-1,3-dienyl)-3-methyl-7-oxo-4-thia-1-aza-bicyclo[3.2.0]heptan-2-carbonsaeure benzhydrylester wird in Analogie zu Beispiel 4, Methode B, aus 815 mg (2.14 mmol) (1E,3E)-(2S,3S,5R)-3-(4-Formyl-buta-1,3-dienyl)-3-methyl-7-oxo-4-thia-1-aza-bicyclo[3.2.0]heptan-2-carbonsäure benzhydrylester hergestellt. Das verbleibende Oel wird ueber Kieselgel (Korngrösse 0.040-0.063 mm) mit Essigester : n-Hexan 1:2 als Laufmittel gereinigt.
Ausbeute: 820 mg (89%) Isomerengemisch, E:Z 2:3
Spektroskopische Daten des Gemisches:
IR (Film): 2215, 1780, 1745, 1202, 989 cm⁻¹
MS: (M+NH₄⁺) 448.5

### (b) (1E,3E) und (1E,3Z)-(2S,3S,5R)-3-(4-Cyano-buta-1,3-dienyl)-3-methyl-4,4,7 -trioxo-4-thia-1-aza-bicyclo[3.2.0]heptan-2-carbonsaeure benzhydrylester

Gemaess Beispiel 8 werden 770 mg (1.79 mmol) (1E,3E) und (1E,3Z)-(2S,3S,5R)-3-(4-Cyano-buta-1,3-dienyl)-3-methyl-7-oxo-4-thia-1-aza-bicyclo[3.2.0]heptan-2-carbonsaeure benzhydrylester oxidiert. Die beiden Isomere können chromatographisch ueber Kieselgel (Korngroesse 0.040-0.063 mm) mit Essigester : n-Hexan 9:16 als Laufmittel getrennt werden. Als erste Komponente wird das Z-Isomer eluiert.
Ausbeute: **Z**: 430 mg (52%) farbloses Oel
**E**: 280 mg (34%) farbloser Schaum
Spektroskopische Daten **E-Isomer**:
MS: (M+NH₄)⁺ 480.5
IR (KBr): 2219, 1800, 1756, 1329, 1191, 994 cm⁻¹
Spektroskopische Daten **Z-Isomer**:
MS: (M+NH₄)⁺ 435.3
IR (KBr): 2210, 1800, 1756, 1330, 1192, 993 cm⁻¹

### (c)(1E,3E)-(2S,3S,5R)-3-(4-Cyano-buta-1,3-dienyl)-3-methyl-4,4,7-trioxo-4-thia-1-aza-bicyclo[3.2.0]heptan-2-carbonsaeure Natriumsalz

In Analogie zu Beispiel 8 werden 250 mg (0.54 mmol) (1E,3E)-(2S,3S,5R)-3-(4-Cyano-buta-1,3-dienyl)-3-methyl-4,4,7-trioxo-4-thia-1-aza-bicyclo[3.2.0]heptan-2-carbonsaeure benzhydrylester entschuetzt.
Ausbeute: 55 mg (41%) gelbliches Lyophilisat
IR (KBr): 2221, 1781, 1631, 1397, 1319, 1139, 992 cm⁻¹
MS: (M+Na)⁺ 341.2

### (1E,3Z) -(2S,3S,5R)-3-(4-Cyano-buta-1,3-dienyl)-3-methyl-4,4,7-trioxo-4-thia-1-aza-bicyclo[3.2.0]heptan-2-carbonsaeure Natriumsalz

In Analogie zu Beispiel 8 werden 230 mg (0.49 mmol) (1E,3E)-(2S,3S,5R)-3-(4-Cyano-buta-1,3-dienyl)-3-methyl-4,4,7-trioxo-4-thia-1-aza-bicyclo[3.2.0]heptan-2-carbonsaeure benzhydrylester entschuetzt.
Ausbeute: 84 mg (58%) gelbliches Lyophilisat
IR (KBr): 2219, 1780, 1625, 1396, 1316, 1138, 951 cm⁻¹
MS: (M+H)⁺ 319.3

| Elementaranalyse: C₁₂H₁₁N₂O₅SNa (318.279) | | | |
|---|---|---|---|
| ber. | C 45.28 | H 3.48 | N 8.80 |
| gef.#) | C 45.29 | H 3.63 | N 8.82 |

| | | | |
|---|---|---|---|
| #) wasserfrei gerechnet mit 6.93% Wasser | | | |

### Beispiel 17

### (a) (E)-(2S,3S,5R)-3-(3-Hydroxy-propen-1-yl)-3-methyl-7-oxo-4-thia-1-aza-bicyclo[3.2.0]heptan-2-carbonsaeure benzhydrylester

5.80 g (14.2 mmol) (E)-(2S,3S,5R)-3-(2-Formyl-vinyl)-3-methyl-7-oxo-4-thia-1-aza-bicyclo[3.2.0]heptan-2-carbonsäure benzhydrylester werden unter Argon in 240 ml Toluol geloest und auf 0°C abgekuehlt. Dann werden 14.2 ml (21.3 mmol) einer 20%igen Diisobutylaluminiumhydrid-Loesung (in Toluol) zugetropft und das Kaeltebad entfernt. Man ruehrt bei Baumtemperatur 6 Stunden nach, giesst das Reaktionsgemisch auf 150 ml gesaettigte Ammoniumchloridloesung und extrahiert dreimal mit je 200 ml Methylenchiorid. Die vereinigten organischen Auszuege werden einmal mit 300 ml Wasser sowie gesaettigter Natriumchloridloesung gewaschen, ueber Magnesiumsulfat getrocknet, filtriert und am Rotationsverdampfer vom Loesungsmittel befreit. Das verbleibende gelbe Oel wird ueber Kieselgel (Korngrösse 0.040-0.063 mm) mit Essigester : n-Hexan 9:16 als Laufmittel chromatographiert.
Ausbeute: 1.90 g (32%) farbloses Harz
IR (Film): 3480(br), 1778, 1750, 1202, 1080, 988 cm⁻¹
MS: (M+NH₄)⁺ 427.6

### (c)(E)-(2S,3S,5R)-3-(3-Hydroxy-propen-1-yl)-3-methyl-4,4,7-trioxo-4-thia-1-aza-bicyclo[3.2.0]heptan-2-carbonsaeure Natriumsalz

Gemaess Beispiel 8 werden 500 mg (0.95 mmol) (E)-(2S,3S,5R)-3-Methyl-4,4,7 -trioxo-3-[3-[(R)- und (S)-tetrahydro-pyran-2-yloxyl]-propenyl]-4-thia-1-aza-bicyclo[3.2.0]heptan-2-carbonsaeure benzhydrylester entschuetzt.
Ausbeute: 165 mg (62%) farbloses Lyophilisat
MS: (M-Na)⁻ 274.3
IR (KBr): 3428, 1780, 1622, 1398, 1311, 1192, 1139, 1082 cm⁻¹

| Elementaranalyse: C₁₀H₁₂NO₆SNa (297.257) | | | |
|---|---|---|---|
| ber. | C 40.41 | H 4.07 | N 4.71 |
| gef.#) | C 40.44 | H 4.48 | N 4.79 |

| | | | |
|---|---|---|---|
| #) wasserfrei gerechnet mit 8.73 % Wasser | | | |

### (E)-(2S,3S,5R)-3-Methyl-7-oxo-3-[3-[(R)- und (S)-tetrahydro-pyran-2-yloxy]-propenyl]-4-thia-1-aza-bicyclo[3.2.0]heptan-2-carbonsaeure benzhydrylester

620 mg (1.50 mmol) (E)-(2S,3S,5R)-3-(3-Hydroxy-propenyl)-3-methyl-7-oxo-4-thia-1-aza-bicyclo[3.2.0]heptan-2-carbonsaeure benzhydrylester werden in 30 ml Methylenchlorid geloest und mit 4.5 mg (0.024 mmol) p-Toluolsulfonsaeure-Monohydrat versetzt. Anschliessend werden 0.25 ml (2.70 mmol) 3,4-Dihydro-2H-pyran zugegeben und 1 Stunde weitergeruehrt. Man entfernt das Loesungsmittel am Rotationsverdampfer und chromatographiert ueber Kieselgel (Korngrösse 0.040-0.063 mm) mit Essigester: n-Hexan 9:16 als Laufmittel .
Ausbeute: 700 mg (93%) farbloses Oel, Diastereomerengemisch (1:1)
IR (Film): 3031, 1782, 1749, 1257, 1134, 967 cm⁻¹
MS: (M+NH₄)⁺ 511.6

### (E)-(2S,3S,5R)-3-Methyl-4,4,7-trioxo-3-[3-[(R)- und (S)-tetrahydro-pyran-2-yloxy]-propenyl]-4-thia-1-aza-bicyclo[3.2.0]heptan-2-carbonsaeure benzhydrylester

Gemaess Beispiel 8 werden 585 mg (1.18 mmol) (E)-(2S,3S,5R)-3-Methyl-7-oxo-3-[3-[(R)- und (S)-tetrahydro-pyran-2-yloxy]-propenyl]-4-thia-1-aza-bicyclo[3.2.0]heptan-2-carbonsaeure benzhydrylester oxidiert. Man chromatographiert ueber Kieselgel (Korngrösse 0.040-0.063 mm) mit Essigester : n-Hexan 9:16 als Laufmittel .
Ausbeute: 525 mg (84%) farbloser Schaum, Diastereomerengemisch (1:1)
IR (KBr): 1800, 1756, 1327, 1189, 1141, 969 cm⁻¹
MS: (M+NH₄)⁺ 543.5

### Beispiel 18

### (a) (E)-(2S,3S,5R)-3-[3-(2-Chlor-acetylaminocarbonyloxy)-propenyl]-3-methyl-7-oxo-4-thia-1-aza-bicyclo[3.2.0]heptan-2-carbonsaeure benzhydrylester

300 mg (0.73 mmol) (E)-(2S,3S,5R)-3-(3-Hydroxy-propenyl)-3-methyl-7-oxo-4-thia-1-aza-bicyclo[3.2.0]heptan-2-carbonsaeure benzhydrylester werden unter Argon in 10 ml Tetrahydrofuran geloest und mit 90 µl (1.05 mmol) Chloracetylisocyanat versetzt. Man ruehrt 3 Stunden nach, rotiert ein und chromatographiert ueber Kieselgel (Korngrösse 0.040-0.063 mm) mit Essigester: n-Hexan 9:16 als Laufmittel .
Ausbeute: 300 mg (77%) farbloser Schaum
IR (KBr): 3300(br), 1779, 1753, 1730, 1495, 1203 cm⁻¹
MS: (M+NH₄)⁺ 546.4

### (b) (E)-(2S,3S,5R)-3-[3-(2-Chlor-acetylaminocarbonyloxy)-propenyl]-3-methyl-4,4,7-trioxo-4-thia-1-aza-bicyclo[3.2.0]heptan-2-carbonsaeure benzhydrylester

Analog Beispiel 8 werden 295 mg (0.57 mmol) (E)-(2S,3S,5R)-3-[3-(2-Chloracetylaminocarbonyloxy)-propenyl]-3-methyl-7-oxo-4-thia-1-aza-bicyclo[3.2.0]heptan-2-carbonsaeure benzhydrylester oxidiert. Man chromatographiert ueber Kieselgel (Korngrösse 0.040-0.063 mm) mit Essigester : n-Hexan 9:16 als Laufmittel .
Ausbeute: 216 mg (68%) farbloser Schaum
IR (KBr): 3413(br), 1798, 1758, 1730, 1705, 1326, 1198, 991 cm⁻¹
MS: (M+NH₄)⁺ 578.4

### (c) (E)-(2S,3S,5R)-3-[3-(2-Chlor-acetylaminocarbonyloxy)-propenyl]-3-methyl-4,4,7-trioxo-4-thia-1-aza-bicyclo[3.2.0]heptan-2-carbonsaeure Natriumsalz

195 mg (0.35 mmol) (E)-(2S,3S,5R)-3-[3-(2-Chlor-acetylaminocarbenyloxy)-propenyl]-3-methyl-4,4,7-trioxo-4-thia-1-aza-bicyclo[3.2.0]heptan-2-carbonsaeure benzhydrylester werden in Analogie zu Beispiel 8 entschuetzt und in das entsprechende Natriumsalz ueberfuehrt.
Ausbeute: 60 mg (49%) farbloses Lyophilisat
MS: (M-COCH₂Cl-Na)⁻ 317.3
IR (KBr): 3435, 1781, 1721, 1622, 1533, 1396, 1314, 1192, 1139 cm⁻¹
¹H-NMR (250MHz, D₂O): δ[ppm] = 1.61(s,3H), 3.42(dd,1H,J=16Hz, 1.6Hz, 6-H), 3.69(dd, 1H, J=16Hz, 4.0Hz, 6-H), 4.43(s,2H,CH₂Cl), 4.49(s,1H,2-H), 4.84(d,2H,J=4.4Hz,CH₂O), 5.08(dd,J=1.6Hz, 4.0Hz, 5-H), 6.08(d(br),1H,J=16Hz,=CH), 6.22(dt,1H,J=16Hz, 4.4Hz, =CH).

### Beispiel 19

### (E)-(2S,3S,5R)-3-(Carbamoyloxy-propenyl)-3-methyl-4,4,7-trioxo-4-thia-1-aza-bicyclo[3.2.0]heptan-2-carbonsaeure benzhydrylester

450 mg (0.80 mmol) (**E**)-(2S,3S,5R)-3-[3-(2-Chlor-acetylaminocarbonyloxy)-propenyl]-3-methyl-4,4,7-trioxo-4-thia-1-aza-bicyclo[3.2.0]heptan-2-carbonsaeure benzhydrylester werden unter Argon in 5 ml Tetrahydrofuran geloest und mit 1.7 ml Methanol versetzt. Zu dieser Loesung gibt man 135 mg (1.61 mmol) Natriumhydrogencarbonat in 2.8 ml Wasser. Nach beendeter Reaktion (DC-Kontrolle) wird das Loesungsmittelgemisch am Rotationsverdampfer entfernt, das verbleibende gelbe Oel in 15 ml Essigester sowie 15 ml gesaettigter Kochsalzloesung aufgenommen, ausgeschuettelt und die Phasen getrennt. Die Wasserphase wird noch zweimal mit je 15 ml Essigester nachextrahiert und die vereinigten organischen Auszuege einmal mit 15 ml gesaettigter Kochsalzloesung gewaschen. Man trocknet ueber Magnesiumsulfat, engt am Rotationsverdampfer ein und chromatographiert das verbleibende Oel ueber Kieselgel (Korngrösse 0.040-0.063 mm) mit Essigester : n-Hexan 2:1 als Laufmittel .
Ausbeute: 200 mg (78%) farbloser Schaum
IR (KBr): 3481, 1799, 1749, 1731, 1601, 1329, 1191, 996 cm⁻¹
MS: (M+NH₄)⁺ 484

### (c) (E)-(2S,3S,5R)-3-(Carbamoyloxy-propenyl)-3-methyl-4,4,7-trioxo-4-thia-1-aza-bicyclo[3.2.0]heptan-2-carbonsaeure Natriumsalz

190mg (0.60 mmol) (E)-(2S,3S,5R)-3-(Carbamoyloxypropenyl)-3-methyl-4,4,7-trioxo-4-thia-1-aza-bicyclo[3.2.0]heptan-2-carbonsaeure benzhydrylester werden in Analogie zu Beispiel 8 entschuetzt.
Ausbeute: 82 mg (62%) farbloses Lyophilisat
¹H-NMR (250MHz, D₂O): δ[ppm] = 1.61(s,3H), 3.42(dd,1H,J=17Hz,1.6Hz,6-H), 3.69(dd,1H,J=17Hz,4Hz,6-H), 4.48(s,1H,2-H), 4.70(d,2H,J=4.6Hz,CH₂), 5.08(dd,1H,J=4Hz, 1.6Hz,5-H), 5.97(d,1H,J=16Hz,=CH), 6.19(dt,1H,J=16Hz, 4.6Hz,=CH).

### Beispiel 20

### (a) (2S,3S,5R)-1-[3-(2-Benzhydryloxycarbonyl-3-methyl-7-oxo-4-thia-1-aza-bicyclo[3.2.0]heptan-3-yl)-allyl]-pyridinium trifluormethansulfonat

410 mg (1.0 mmol) (E)-(2S,3S,5R)-3-(3-Hydroxy-propenyl)-3-methyl-7-oxo-4-thia-1-aza-bicyclo[3.2.0]heptan-2-carbonsaeure benzhydrylester werden in 6 ml Methylenchlorid geloest, auf -40°C gekuehlt und mit 250 µl (1.5 mmol) Trifluormethan-sulfonsaeureanhydrid versetzt. Nach 5 Minuten werden 200 µl (2.50 mmol) Pyridin zugegeben, eine weitere Stunde bei gleicher Temperatur nachgeruehrt und anschliessend das Kaeltebad entfernt. Bei Raumtemperatur wird das Loesungsmittel am Rotationsverdampfer entfernt, das Reaktionsgemisch in 20 ml Methylenchlorid aufgenommen und zweimal mit je 10 ml gesaettigter Kochsalzloesung gewaschen. Man trocknet ueber Magnesiumsulfat, filtriert und engt am Rotationsverdampfer ein. Das verbleibend rote Harz wird ohne weitere Reinigung oxidiert.
Ausbeute: 600 mg ( 97%) rotes Harz
MS: (M⁺) 471.6
IR (KBr): 1777, 1743, 1630, 1160, 987 cm⁻¹

### (b) (2S,3S,5R)-1-[3-(2-Benzhydryloxycarbonyl-3-methyl-4,4,7-trioxo-4-thia-1-aza-bicyclo[3.2.0]heptan-3-yl)-allyl]-pyridinium trifluormethansulfonat

In Analogie zu Beispiel 8 werden 600 mg (0.97 mmol) (2S,3S,5R)-1-[3-(2-Benzhydryloxycarbonyl-3-methyl-7-oxo-4-thia-1-aza-bicyclo[3.2.0]heptan-3-yl)-allyl]-pyridinium trifluormethansulfonat oxidiert.
Ausbeute: 440 mg (70%) gruenes Harz
MS: (M⁺) 503.3
IR (KBr): 1797, 1753, 1632, 1167 cm⁻¹

### (E)-(2S,3S,5R)-3-Methyl-4,4,7-trioxo-3-(3-pyridin-1-ylio-propenyl)-4-thia-1-aza-bicyclo[3.2.0]heptan-2-carboxylat

Die Loesung von 440 mg (0.67 mmol) (2S,3S,5R)-1-[3-(2-Benzhydryloxycarbonyl-3-methyl-4,4,7-trioxo-4-thia-1-aza-bicyclo[3.2.0]heptan-3-yl)-allyl]-pyridinium trifluormethansulfonat in 3 ml m-Kresol wird 1.5 Stunden bei 50°C geruehrt. Man verduennt bei Raumtemperatur mit 10 ml Isobutylmethylketon und extrahiert dreimal mit je 8 ml Wasser. Die vereinigten waessrigen Auszuege werden noch zweimal mit je 10 ml Isobutylmethylketon gewaschen, ueber einen Papierfilter filtriert und lyophilisiert. Das braune Lyophilisat wird anschliessend über polymeres hydrophobes Gel mit Wasser als Elutionsmittel chromatographiert.
Ausbeute: 125 mg (55%) beiges Lyophilisat
IR (KBr): 1780, 1625, 1485, 1372, 1312, 980 cm⁻¹
MS: M+H⁺ 337.4

## Patentansprüche

1. 3β-Alkenyl-penam-Derivate der allgemeinen Formel 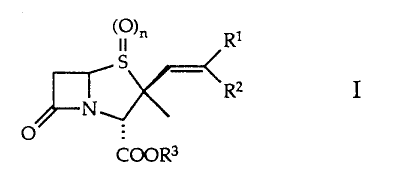 worin
eines von R¹ und R² -COR⁴, -CN, -CH₂R⁵, Halogen, -CH=CHR⁶ oder Q und das andere Wasserstoff oder **C**_{**1**}**-C**_{**7**}-Alkyl oder beide zusammen einen γ-Lactamring,
R³ Wasserstoff, **C**_{**1**}**-C**_{**7**}-Alkyl, **Benzyl, Benzhydryl, p-Methoxybenzyl, p-Nitrobenzyl,** Allyl oder einen in vivo abspaltbaren Rest, **der eine Estergruppe enthält, wie -CH**_{**2**}**OOCC(CH**_{**3**}**)**_{**3**}**,** 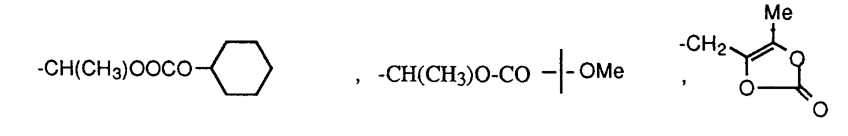 **oder -CH(CH**_{**3**}**)OCO-CH**_{**3**}**.**
R⁴ Wasserstoff, **C**_{**1**}**-C**_{**7**}-Alkyl, **C**_{**1**}**-C**_{**7**}-Alkoxy, Benzyloxy, Amino, **C**_{**1**}**-C**_{**7**}-Alkylamino oder **C**_{**1**}**-C**_{**7**}**-**Alkyl **C**_{**1**}**-C**_{**7**}-Alkoxyamino,
R⁵ Hydroxy, -OCONHR⁷, -OCONH₂ oder einen fünf- oder sechsgliedrigen, N,S und/oder O enthaltenden hetero-aromatischen, über ein Stickstoffatom verknüpften Ring,
R⁶ -CN oder -CHO,
R⁷ -COCH₂Cl,
Q **2-Pyridyl, 1-Methyl-pyridin-2-ylio, 1,3-Thiazol-2-yl, 1,2,4-Oxadiazol-3-yl** und
n 0, 1 oder 2 bedeuten,
sowie die pharmazeutisch verträglichen Salze dieser Verbindungen.

2. Verbindungen gemäss Anspruch 1, worin
eines von R¹ und R² -COR⁴, -CN, oder CH₂R⁵ und das andere Wasserstoff,
oder **C**_{**1**}**-C**_{**7**}-Alkyl,
R³ Wasserstoff, **C**_{**1**}**-C**_{**7**}-Alkyl, **Benzyl, Benzhydryl, p-Methoxybenzyl, p-Nitrobenzyl,** Allyl oder einen in vivo abspaltbaren Rest, **der eine Estergruppe enthält, wie -CH**_{**2**}**OOCC(CH**_{**3**}**)**_{**3**}**,** 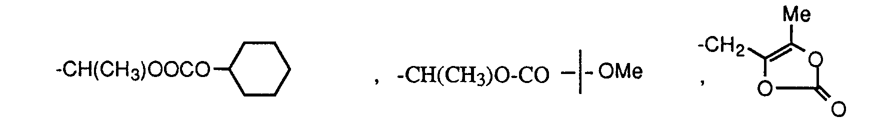 **oder -CH(CH**_{**3**}**)OCO-CH**_{**3**}**.,**
R⁴ Wasserstoff, **C**_{**1**}**-C**_{**7**}-Alkyl, **C**_{**1**}**-C**_{**7**}-Alkoxy, Benzyloxy, Amino oder **C**_{**1**}**-C**_{**7**}-Alkylamino,
R⁵ Hydroxy oder -OCONH₂ und
n 0, 1 oder 2 bedeuten.

3. Verbindungen gemäss einem der Ansprüche 1 oder 2, worin R¹ CN, Halogen oder COR⁴, R² Wasserstoff und R⁴ Methyl, **C**_{**1**}**-C**_{**7**}-Alkoxy, Benzyloxy oder Amino bedeuten.

4. Verbindungen gemäss einem der Ansprüche 1 bis 3, worin n 0 oder 2 bedeutet.

5. Verbindungen gemäss einem der Ansprüche 1 bis 4, worin R³ Wasserstoff oder Benzhydryl bedeutet.

6. **Die Verbindung nach Anspruch 1** (E/Z)-(2S,3S,5R)-3-(2-Cyanoethenyl)-3-methyl-7-oxo-4-thia-1-aza-bicyclo[3.2.0]heptan-2-carbonsäure-benzhydrylester.

7. **Die Verbindung nach Anspruch 1** (E)-(2S,3S,5R)-3-(2-Cyanoethenyl)-3-methyl-4,4,7-trioxo-4-thia-1-aza-bicyclo[3.2.0]heptan-2-carbonsäure-benzhydrylester.

8. **Die Verbindung nach Anspruch 1** (Z)-(2S,3S,5R)-3-(2-Cyanoethenyl)-3-methyl-4,4,7-trioxo-4-thia-1-aza-bicyclo[3.2.0]heptan-2-carbonsäure-benzhydrylester.

9. **Die Verbindung nach Anspruch 1** (E)-(2S,3S,5R)-3-(2-Cyanoethenyl)-3-methyl-4,4,7-trioxo-4-thia-1-aza-bicyclo[3.2.0]heptan-2-carbonsäure-Natriumsalz.

10. **Die Verbindung nach Anspruch 1** (Z)-(2S,3S,5R)-3-(2-Cyanoethenyl)-3-methyl-4,4,7-trioxo-4-thia-1-aza-bicyclo[3.2.0]heptan-2-carbonsäure-Natriumsalz.

11. **Die Verbindung nach Anspruch 1** (E)-(2S,3S,5R)-3-(2-Chlor-vinyl)-3-methyl-4,4,7-trioxo-4-thia-1-aza-bicyclo[3.2.0]heptan-2-carbonsäure-Natriumsalz.

12. **Die Verbindung nach Anspruch 1** (E)-(2S,3S,5R)-3-Methyl-3-(3-oxo-but-1-enyl)-4,4,7-trioxo-4-thia-1-aza-bicyclo[3.2.0]heptan-2-carbonsäure-Natriumsalz.

13. **Die Verbindungen nach Anspruch 1** (E/Z)-(2S,3S,5R)-3-(2-Carbamoyl-vinyl)-3-methyl-7-oxo-4-thia-1-aza-bicyclo[3.2.0]heptan-2-carbonsäure-benzhydrylester,
(E)-(2S,3S,5R)-3-(2-Carbamoyl-vinyl)-3-methyl-4,4,7-trioxo-4-thia-1-aza-bicyclo[3.2.0]heptan-2-carbonsäure-benzhydrylester,
(Z)-(2S,3S,5R)-3-(2-Carbamoyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Carbamoyl-vinyl)-3-methyl-4,4,7-trioxo-4-thia-1-aza-bicyclo[3.2.0]heptan-2-carbonsäure-Natriumsalz,
(Z)-(2S,3S,5R)-3-(2-Carbamoyl-vinyl)-3-methyl-4,4,7-trioxo-4-thia-1-aza-bicyclo[3.2.0]heptan-2-carbonsäure-Natriumsalz,
(E)-(2S,3S,5R)-3-(2-Ethoxycarbonyl-vinyl)-3-methyl-7-oxo-4-thia-1-aza-bicyclo[3.2.0]heptan-2-carbonsäure-benzhydrylester,
(E)-(2S,3S,5R)-3-(2-Ethoxycarbonyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Ethoxycarbonyl-vinyl)-3-methyl-4,4,7-trioxo-4-thia-1-aza-bicyclo[3.2.0]heptan-2-carbonsäure-Natriumsalz,
(E)-(2S,3S,5R)-3-(2-Benzyloxycarbonyl-vinyl)-3-methyl-7-oxo-4-thia-1-aza-bicyclo[3.2.0]heptan-2-carbonsäure-benzhydrylester,
(E)-(2S,3S,5R)-3-(2-Benzyloxycarbonyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Benzyloxycarbonyl-vinyl)-3-methyl-4,4,7-trioxo-4-thia-1-aza-bicyclo[3.2.0]heptan-2-carbonsäure-Natriumsalz.

14. Verbindungen der allgemeinen Formel 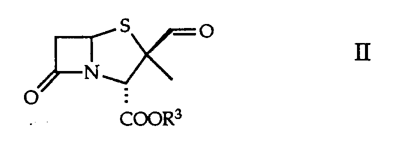 worin R³ die in Anspruch 1 angegebene Bedeutung hat.

15. **Die Verbindung nach Anspruch 14** (2S,3R,5R)-3-Formyl-3-methyl-7-oxo-4-thia-1-azabicyclo[3.2.0]heptan-2-carbonsäure-benzyhydrylester.

16. Verbindungen gemäss einem der Ansprüche 1 bis 13 zur Anwendung als therapeutische Wirkstoffe.

17. Verbindungen gemäss einem der Ansprüche 1 bis 13 zur Anwendung als β-Lactamasehemmer.

18. Verfahren zur Herstellung von Verbindungen gemäss einem der Ansprüche 1 bis 13, dadurch gekennzeichnet, dass man
a) eine Verbindung der allgemeinen Formel 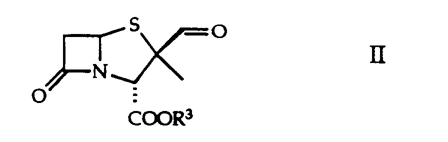 worin R³ die in Anspruch 1 angegebene Bedeutung hat, mit einer Verbindung der allgemeinen Formel 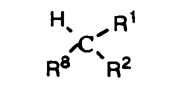 worin R¹ und R² die in Anspruch 1 angegebene Bedeutung haben
und R⁸ = P^{⊕}(Aryl)₃, PO(OAlkyl)₂, Si(Alkyl)₃ oder Halogen bedeutet, in Gegenwart eines Aktivierungsmittels umsetzt, oder
b) eine Verbindung der allgemeinen Formel I, worin n 0 bedeutet, oxydiert, oder
c) eine Verbindung der allgemeinen Formel I, worin R³ von Wasserstoff verschieden ist, in die entsprechende freie Säure überführt und
d) erwünschtenfalls eine saure Verbindung der Formel I in ein pharmazeutisch annehmbares Salz überführt.

19. Arzneimittel, enthaltend eine Verbindung gemäss einem der Ansprüche 1 bis 13.

20. β-Lactamase hemmende Arzneimittel, enthaltend eine Verbindung gemäss einem der Ansprüche 1 bis 13.

21. Arzneimittel gemäss Anspruch 19 oder 20, enthaltend zusätzlich ein β-Lactam-Antibiotikum.

22. Arzneimittel gemäss Anspruch 21, enthaltend ein Penicillin, Cephalosporin, Peneme oder Carbapeneme als β-Lactam-Antibiotikum.

23. Arzneimittel gemäss Anspruch 22, enthaltend Piperacillin, Mezlocillin, Azlocillin, Apalcillin, Benzylpenicillin, Phenoxymethyl-Penicillin, Carbenicillin, Methicillin, Propicillin, Ticartillin, Ampicillin, Amoxicillin, Mecillinam, Ceftriaxon, Ceftazidim, Cefetamet, Cefetamet-Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, Cefpiron, Cefepim, Imipenem oder Meropenem oder ein pharmazeutisch verträgliches Salz einer dieser Verbindungen.

24. Arzneimittel gemäss Anspruch 22, enthaltend Ceftriaxon oder eines seiner pharmazeutisch verträglichen Salze.

25. Arzneimittel gemäss einem der Ansprüche 21 bis 24 als Kombinationspräparat zur gleichzeitigen, getrennten oder zeitlich abgestuften Anwendung in der antibakteriellen Therapie.

26. Verwendung von Verbindungen gemäss einem der Ansprüche 1 bis 13 zur Herstellung eines Medikaments für die Bekämpfung oder Verhütung von Krankheiten.

27. Verwendung von Verbindungen gemäss einem der Ansprüche 1 bis 13 zur Herstellung eines Medikaments für die Bekämpfung oder Verhütung von bakteriellen Infektionen.

28. Verwendung von Verbindungen gemäss einem der Ansprüche 1 bis 13 zur Herstellung von antibakteriell wirksamen Mitteln.

## Claims

1. 3β-Alkenyl-penam derivatives of the general formula 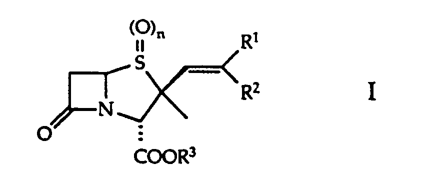 wherein
one of R¹ and R² signifies -COR⁴, -CN, -CH₂R⁵, halogen, -CH=CHR⁶ or Q and the other signifies hydrogen or C₁-C₇-alkyl or both together signify a γ-lactam ring,
R³ signifies hydrogen, C₁-C₇-alkyl, benzyl, benzhydryl, p-methoxybenzyl, p-nitrobenzyl, ally or a residue which is cleavable in vivo and which contains an ester group, such as -CH₂OOCC(CH₃)₃, 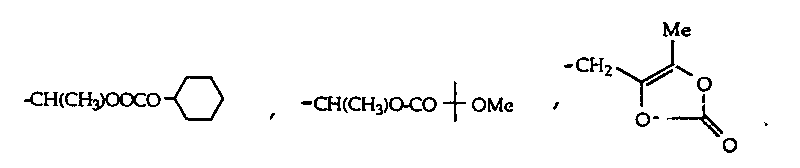 or -CH(CH₃)OCO-CH₃,
R⁴ signifies hydrogen, C₁-C₇-alkyl, C₁-C₇-alkoxy, benzyloxy, amino, C₁-C₇-alkylamino or C₁-C₇-alkyl-C₁-C₇-alkoxyamino,
R⁵ signifies hydroxy, -OCONHR⁷, -OCONH₂ or a five- or six-membered hetero-aromatic ring which contains N,S and/or O and which is linked via a nitrogen atom,
R⁶ signifies -CN or -CHO,
R⁷ signifies -COCH₂Cl,
Q signifies 2-pyridyl, 1-methyl-pyridin-2-ylio, 1,3-thiazol-2-yl, 1,2,4-oxadiazol-3-yl and
n signifies 0, 1 or 2,
as well as pharmaceutically compatible salts of these compounds.

2. Compounds according to claim 1,
wherein
one of R¹ and R² signifies -COR⁴, -CN or -CH₂R⁵ and the other signifies hydrogen or C₁-C₇-alkyl,
R³ signifies hydrogen, C₁-C₇-alkyl, benzyl, benzhydryl, p-methoxybenzyl, p-nitrobenzyl, allyl or a residue which is cleavable in vivo and which contains an ester group, such as -CH₂OOCC(CH₃)₃, 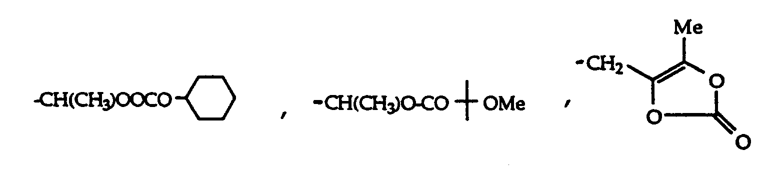 or -CH(CH₃)OCO-CH₃,
R⁴ signifies hydrogen, C₁-C₇-alkyl, C₁-C₇-alkoxy, benzyloxy, amino or C₁-C₇-alkylamino,
R⁵ signifies hydroxy or -CONH₂ and
n signifies 0, 1 or 2.

3. Compounds according to claim 1 or 2, wherein R¹ signifies CN, halogen or COR⁴, R² signifies hydrogen and R⁴ signifies methyl, C₁-C₇-alkoxy, benzyloxy or amino.

4. Compounds according to any one of claims 1 to 3, wherein n signifies 0 or 2.

5. Compounds according to any one of claims 1 to 4, wherein R³ signifies hydrogen or benzhydryl.

6. The compound according to claim 1, benzhydryl (E/Z)-(2S,3S,5R)-3-(2-cyanoethenyl)-3-methyl-7-oxo-4-thia-1-aza-bicyclo[3.2.0]heptane-2-carboxylate.

7. The compound according to claim 1, benzhydryl (E)-(2S,3S,5R)-3-(2-cyanoethenyl)-3-methyl-4,4,7-trioxo-4-thia-1-aza-bicyclo[3.2.0]heptane-2-carboxylate.

8. The compound according to claim 1, benzhydryl (Z)-(2S,3S,SR)-3-(2-cyanoethenyl)-3-methyl-4,4,7-trioxo-4-thia-1-aza-bicyclo[3.2.0]heptane-2-carboxylate.

9. The compound according to claim 1, sodium (E)-(2S,3S,5R)-3-(2-cyanoethenyl)-3-methyl-4,4,7-trioxo-4-thia-1-aza-bicyclo[3.2.0]heptane-2-carboxylate.

10. The compound according to claim 1, sodium (Z)-(2S,3S,5R)-3-(2-cyanoethenyl)-3-methyl-4,4,7-trioxo-4-thia-1-aza-bicyclo[3.2.0]heptane-2-carboxylate.

11. The compound according to claim 1, sodium (E)-(2S,3S,5R)-3-(2-chloro-vinyl)-3-methyl-4,4,7-trioxo-4-thia-1-aza-bicyclo[3.2.0]heptane-2-carboxylate.

12. The compound according to claim 1, sodium (E)-(2S,3S,5R)-3-methyl-3-(3-oxo-but-1-enyl)-4,4,7-trioxo-4-thia-1-aza-bicyclo[3.2.0]heptane-2-carboxylate.

13. The compounds according to claim 1, benzhydryl (E/Z)-(2S,3S,5R)-3-(2-carbamoyl-vinyl)-3-methyl-7-oxo-4-thia-1-aza-bicyclo[3.2.0]heptane-2-carboxylate,
benzhydryl (E)-(2S,3S,5R)-3-(2-carbamoyl-vinyl)-3-methyl-4,4,7-trioxo-4-thia-1-aza-bicyclo[3.2.0]heptane-2-carboxylate,
benzhydryl (Z)-(2S,3S,5R)-3-(2-carbamoyl-vinyl)-3-methyl-4,4,7-trioxo-4-thia-1-azabicyclo[3.2.0]heptane-2-carboxylate,
sodium (E)-(2S,3S,5R)-3-(2-carbamoyl-vinyl)-3-methyl-4,4,7-trioxo-4-thia-1-aza-bicyclo[3.2.0]heptane-2-carboxylate,
sodium (Z)-(2S,3S,5R)-3-(2-carbamoyl-vinyl)-3-methyl-4,4,7-trioxo-4-thia-1-aza-bicyclo[3.2.0]heptane-2-carboxylate,
benzhydryl (E)-(2S,3S,5R)-3-(2-ethoxycarbonyl-vinyl)-3-methyl-7-oxo-4-thia-1-aza-bicyclo[3.2.0]heptane-2-carboxylate,
benzhydryl (E)-(2S,3S,5R)-3-(2-ethoxycarbonyl-vinyl)-3-methyl-4,4,7-trioxo-4-thia-1-aza-bicyclo[3.2.0]heptane-2-carboxylate,
sodium (E)-(2S,3S,5R)-3-(2-ethoxycarbonyl-vinyl)-3-methyl-4,4,7-trioxo-4-thia-1-aza-bicyclo[3.2.0]heptane-2-carboxylate,
benzhydryl (E)-(2S,3S,5R)-3-(2-benzyloxycarbonyl-vinyl)-3-methyl-7-oxo-4-thia-1-aza-bicyclo[3.2.0]heptane-2-carboxylate,
benzhydryl (E)-(2S,3S,5R)-3-(2-benzyloxycarbonyl-vinyl)-3-methyl-4,4,7-trioxo-4-thia-1-aza-bicyclo[3.2.0]heptane-2-carboxylate,
sodium (E)-(2S,3S,5R)-3-(2-benzyloxycarbonylvinyl)-3-methyl-4,4,7-trioxo-4-thia-1-aza-bicyclo[3.2.0]heptane-2-carboxylate.

14. Compounds of the general formula 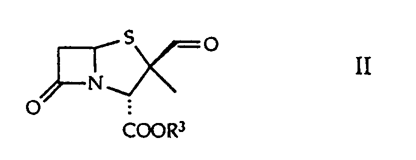 wherein R³ has the significance given in claim 1.

15. The compound according to claim 14, benzhydryl (2S,3R,5R)-3-formyl-3-methyl-7-oxo-4-thia-1-azabicyclo-[3.2.0]heptane-2-carboxylate.

16. Compounds according to any one of claims 1 to 13 for use as therapeutically active substances.

17. Compounds according to any one of claims 1 to 13 for use as β-lactamase inhibitors.

18. A process for the manufacture of compounds according to any one of claims 1 to 13, characterized by
a) reacting a compound of the general formula 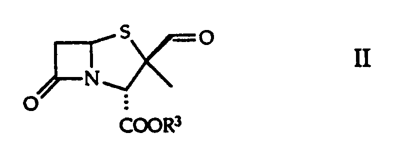 wherein R³ has the significance given in claim 1, with a compound of the general formula 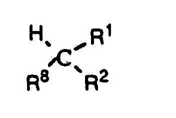 wherein R¹ and R² have the significance given in claim 1
and R⁸ signifies P^{⊕}(aryl)₃, PO(Oalkyl)₂, Si(alkyl)₃ or halogen,
in the presence of an activating agent, or
b) oxidizing a compound of general formula I in which n signifies 0, or
c) converting a compound of general formula I in which R³ is different from hydrogen into the corresponding free acid, and
d) if desired, converting an acidic compound of formula I into a pharmaceutically acceptable salt.

19. A medicament containing a compound according to any one of claims 1 to 13.

20. A β-lactamase-inhibiting medicament containing a compound according to any one of claims 1 to 13.

21. A medicament according to claim 19 or 20 which additionally contains a β-lactam antibiotic.

22. A medicament according to claim 21 containing a penicillin, cephalosporin, penem or cabapenem as the β-lactam antibiotic.

23. A medicament according to claim 22 containing piperacillin, mezlocillin, azlocillin, apalcillin, benzylpenicillin, phenoxymethylpenicillin, carbenicillin, methicillin, propicillin, ticarcillin, ampicillin, amoxicillin, mecillinam, ceftriaxone, ceftazidime, cefetamet, cefetamet pivoxil, cefotaxime, cefmenoxime, ceftizoxime, cefuroxime, cephaloridine, cephalotin, cefazolin, cephalexin, cefoxitin, cephacetrile, cefamandole, cephapirin, cephradine, cephaloglycine, cefpirone, cefepime, imipenem or meropenem or a pharmaceutically compatible salt of one of these compounds.

24. A medicament according to claim 22 containing ceftriaxone or one of its pharmaceutically compatible salts.

25. A medicament according to any one of claims 21 to 24 as a combination preparation for the simultaneous, separate or chronologically stepwise use in antibacterial therapy.

26. The use of compounds according to any one of claims 1 to 13 for the manufacture of a medicament for the control or prevention of illnesses.

27. The use of compounds according to any one of claims 1 to 13 for the manufacture of a medicament for the control or prevention of bacterial infections.

28. The use of compounds according to any one of claims 1 to 13 for the manufacture of antibacterially-active medicaments.

## Revendications

1. Dérivés de 3-β-alcényl-pénam de formule générale : 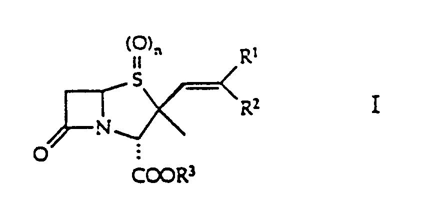 dans laquelle :
l'un des radicaux R¹ et R² est -COR⁴, - CN, -CH₂R⁵, un halogène, -CH₂=CHR⁶ ou Q, et l'autre est un hydrogène ou un groupe alkyle en C₁-C₇, ou les deux forment ensemble un noyau γ-lactame,
R³ est un hydrogène ou un groupe alkyle on C₁-C₇, benzyle, benzhydryle, p-méthoxybenzyle, p-nitrobenzyle, allyle, ou un résidu éliminable in vivo contenant un groupe ester, tel que -CH₂OOCC(CH₃)₃, 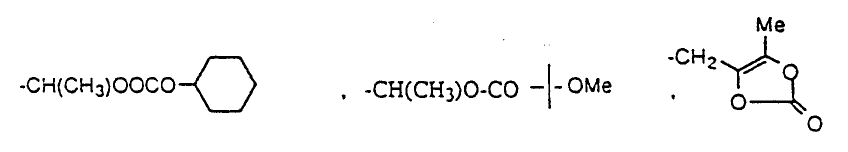 ou -CH(CH₃)OCO-CH₃,
R⁴ est un hydrogène ou un groupe alkyle en C₁-C₇, alcoxy en C₁-C₇, benzyloxy, amino, alkylamino en C₁-C₇ ou (alkyle en C₁-C₇)(alcoxy en C₁-C₇)amino,
R⁵ est un groupe hydroxy, -OCONHR⁷, -OCONH₂, ou un noyau à 5 ou 6 chaînons, hétéroaromatique contenant N, S et/ou O, relié par l'intermédiaire d'un atome d'azote,
R⁶ est -CN ou -CHO,
R⁷ est -COCH₂Cl,
Q est un groupe 2-pyridyle, 1-méthylpyridin -2-ylio, 1,3-thiazol -2-yle, 1,2,4-oxadiazol -3-yle, et
n vaut 0, 1 ou 2,
ainsi que les sels, compatibles d'un point de vue pharmaceutique, de ces composés.

2. Composés selon la revendication 1, dans lesquels :
l'un des radicaux R¹ et R² est -COR⁴, -CN ou CH₂R⁵, et l'autre est un hydrogène ou un groupe alkyle en C₁-C₇,
R³ est un hydrogène ou un groupe alkyle en C₁-C₇, benzyle, benzhydryle, p-méthoxybenzyle, p-nitrobenzyle, allyle, ou un résidu éliminable in vivo contenant un groupe ester, tel que -CH₂OOCC(CH₃)₃, 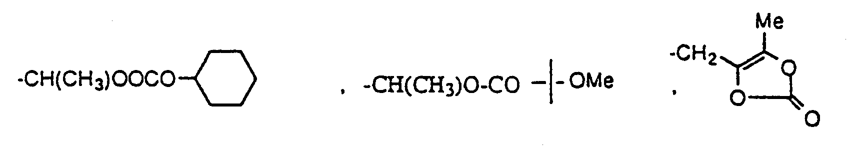 ou -CH(CH₃)OCO-CH₃,
R⁴ est un hydrogène ou un groupe alkyle en C₁-C₇, alcoxy en C₁-C₇, benzyloxy, amino ou alkylamino en C₁-C₇,
R⁵ est un groupe hydroxy ou -OCONH₂, et
n vaut 0, 1 ou 2.

3. Composés selon l'une des revendications 1 ou 2, dans lesquels R¹ est CN, un halogène ou COR⁴, R² est un hydrogène et R⁴ est le groupe méthyle, un groupe alcoxy en C₁-C₇, benzyloxy ou amino.

4. Composés selon l'une des revendications 1 à 3, dans lesquels n vaut 0 ou 2.

5. Composés selon l'une des revendications 1 à 4, dans lesquels R³ est l'hydrogène ou le groupe benzhydryle.

6. Composé selon la revendication 1, qui est l'ester benzhydrylique de l'acide(E/Z)-(2S,3S,5R)-3-(2-cyanoéthényl)-3-méthyl-7-oxo-4-thia-1-aza-bicyclo[3.2.0]-heptane-2-carboxylique.

7. Composé selon la revendication 1, qui est l'ester benzhydrylique de l'acide (E)-(2S,3S,5R)-3-(2-cyanoéthényl)-3-méthyl-4,4,7-trioxo-4-thia-1-aza-bicyclo[3.2.0]heptane-2-carboxylique.

8. Composé selon la revendication 1, qui est l'ester benzhydrylique de l'acide (Z)-(2S,3S,5R)-3-(2-cyanoéthényl)-3-méthyl-4,4,7-trioxo-4-thia-1-aza-bicyclo[3.2.0]heptane-2-carboxylique.

9. Composé selon la revendication 1, qui est le sel de sodium de l'acide (E)-(2S,3S,5R)-3-(2-cyanoéthényl)-3-méthyl-4,4,7-trioxo-4-thia-1-aza-bicyclo[3.2.0]-heptane-2-carboxylique.

10. Composé selon la revendication 1, qui est le sel de sodium de l'acide (Z)-(2S,3S,5R)-3-(2-cyano-éthényl)-3-méthyl-4,4,7-trioxo-4-thia-1-aza-bicyclo-[3.2.0]heptane-2-carboxylique.

11. Composé selon la revendication 1, qui est le sel de sodium de l'acide (E)-(2S,3S,5R)-3-(2-chloro-vinyl)-3-méthyl-4,4,7-trioxo-4-thia-1-aza-bicyclo[3.2.0]heptane-2-carboxylique.

12. Composé selon la revendication 1, qui est le sel de sodium de l'acide (E)-(2S,3S,5R)-3-méthyl-3-(3-oxo-but-1-ényl)-4,4,7-trioxo-4-thia-1-aza-bicyclo-[3.2.0]heptane-2-carboxylique.

13. Composés selon la revendication 1, qui sont :
l'ester benzhydrylique de l'acide (E/Z)-(2S,3S,5R)-3-(2-carbamoylvinyl)-3-méthyl-7-oxo-4-thia-1-aza-bicyclo[3.2.0]heptane-2-carboxylique,
l'ester benzhydrylique de l'acide (E)-(2S,3S,5R)-3-(2-carbamoylvinyl)-3-méthyl-4,4,7-trioxo-4-thia-1-aza-bicyclo[3.2.0]heptane-2-carboxylique,
l'ester benzhydrylique de l'acide (Z)-(2S,3S,5R)-3-(2-carbamoylvinyl)-3-méthyl-4,4,7-trioxo-4-thia-1-aza-bicyclo[3.2.0]heptane-2-carboxylique,
le sel de sodium de l'acide (E)-(2S,3S,5R)-3-(2-carbamoylvinyl)-3-méthyl-4,4,7-trioxo-4-thia-1-aza- bicyclo[3.2.0]heptane-2-carboxylique,
le sel de sodium de l'acide (Z)-(2S,3S,5R)-3-(2-carbamoylvinyl)-3-méthyl-4,4,7-trioxo-4-thia-1-aza-bicyclo[3.2.0]heptane-2-carboxylique,
l'ester benzhydrylique de l'acide (E)-(2S,3S,5R)-3-(2-éthoxycarbonylvinyl)-3-méthyl-7-oxo-4-thia-1-aza-bicyclo[3.2.0]heptane-2-carboxylique,
l'ester benzhydrylique de l'acide (E)-(2S,3S,5R)-3-(2-éthoxycarbonylvinyl)-3-méthyl-4,4,7-trioxo-4-thia-1-aza-bicyclo[3.2.0]heptane-2-carboxylique,
le sel de sodium de l'acide (E)-(2S,3S,5R)-3-(2-éthoxycarbonylvinyl)-3-méthyl-4,4,7-trioxo-4-thia-1-aza-bicyclo[3.2.0]heptane-2-carboxylique,
l'ester benzhydrylique de l'acide (E)-(2S,3S,5R)-3-(2-benzyloxycarbonylvinyl)-3-méthyl-7-oxo-4-thia-1-aza-bicyclo[3.2.0]heptane-2-carboxylique,
l'ester benzhydrylique de l'acide (E)-(2S,3S,5R)-3-(2-benzyloxycarbonylvinyl)-3-méthyl-4,4,7-trioxo-4-thia-1-aza-bicyclo[3.2.0]heptane-2-carboxylique,
le sel de sodium de l'acide (E)-(2S,3S,5R)-3-(2-benzyloxycarbonyivinyl)-3-méthyl-4,4,7-trioxo-4-thia-1-aza-bicyclo[3.2.0]heptane-2-carboxylique.

14. Composés de formule générale : 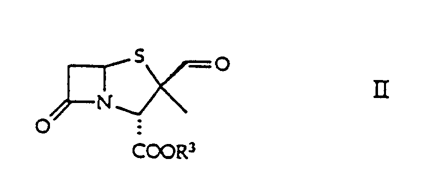 dans laquelle R³ a les significations données dans la revendication 1.

15. Composé selon la revendication 14, qui est l'ester benzhydrylique de l'acide (2S,3R,5R)-3-formyl-3-méthyl-7-oxo-4-thia-1-aza-bicyclo[3.2.0]heptane-2-carboxylique.

16. Composés selon l'une des revendications 1 à 13, pour utilisation en tant que principes actifs thérapeutiques.

17. Composés selon l'une des revendications 1 à 13, pour utilisation en tant qu'inhibiteurs de la β-lactamase.

18. Procédé de préparation de composés selon l'une des revendications 1 à 13, caractérisé en ce que :
a) on fait réagir en présence d'un agent d'activation un composé de formule générale : 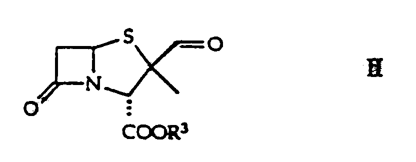 dans laquelle R³ a les significations données dans la revendication 1, avec un composé de formule générale : 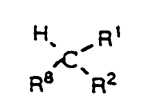 dans laquelle R¹ et R² ont les significations données dans la revendication 1, et R⁸ est un radical P⁺(aryle)₃, PO(O-alkyle)₂, Si(alkyle)₃ ou halogéno, ou bien,
b) on oxyde un composé de formule générale I dans laquelle n vaut 0, ou bien
c) on convertit en l'acide libre correspondant un composé de formule générale I dans laquelle R³ est différent de l'hydrogène, et
d) si on le souhaite, on convertit un composé acide de formule I en un sel acceptable d'un point de vue pharmaceutique.

19. Médicament contenant un composé selon l'une des revendications 1 à 13.

20. Médicament inhibiteur de la β-lactamase, contenant un composé selon l'une des revendications 1 à 13.

21. Médicament selon la revendication 19 ou 20, contenant en outre un antibiotique de type β-lactame.

22. Médicament selon la revendication 21, contenant une pénicilline, une céphalosporine, des pénems ou des carbapénems en tant qu'antibiotique de type β-lactame.

23. Médicament selon la revendication 22, contenant de la pipéracilline, de la mézlocilline, de l'azlocilline, de l'apalcilline, de la benzylpénicilline, de la phénoxyméthylpénicilline, de la carbénicilline, de la méthicilline, de la propicilline, de la ticarcilline, de l'ampicilline de l'amoxicilline, du mécillinam, du ceftriaxon, du ceftazidime, du céfétamet, du céfétametpivoxil, du céfotaxime, du cefménoxime, du ceftizoxime, du céfuroxime, de la céphaloridine, de la céphalotine, de la céfazoline, de la céphalexine, de la céfoxitine, du céphacétril, du céfamandol, de la céphapirine, de la céphradine, de la céphaloglycine, du cefpiron, du céfépime, de l'imipénem ou du méropénem, ou un sel compatible d'un point de vue pharmaceutique de l'un de ces composés.

24. Médicament selon la revendication 22, contenant du ceftriaxon ou l'un de ses sels tolérés du point de vue pharmaceutique.

25. Médicament selon l'une des revendications 21 à 24, en tant que préparation en combinaison pour une utilisation simultanée, séparée ou étagée dans le temps, dans le cadre d'un traitement anti-bactérien.

26. Utilisation de composés selon l'une des revendications 1 à 13 pour préparer un médicament destiné à la maîtrise ou à la prévention de maladies.

27. Utilisation de composés selon l'une des revendications 1 à 13 pour la préparation d'un médicament destiné à la maîtrise ou à la prévention d'infections bactériennes.

28. Utilisation de composés selon l'une des revendications 1 à 13 pour préparer des agents à effet antibactérien.
